(19) Europäisches Patentamt
European Patent Office
Office européen des brevets

(11) Numéro de publication: **0 082 793**
**B1**

(12) # FASCICULE DE BREVET EUROPÉEN

(45) Date de publication du fascicule du brevet: **10.05.89**

(21) Numéro de dépôt: **82402378.2**

(22) Date de dépôt: **23.12.82**

(51) Int. Cl.⁴: **C 07 H 7/02**, A 61 K 31/00, G 01 N 33/48, C 07 H 3/04, C 07 H 3/06, C 07 B 59/00, C 07 H 9/04, C 07 H 15/18, C 07 H 13/04, C 12 N 11/00

(54) **Nouveaux dérivés à structure acide uronique, leur préparation et leurs applications biologiques.**

(30) Priorité: **23.12.81 FR 8124132**
**15.01.82 FR 8200621**
**01.02.82 FR 8201575**
**28.05.82 FR 8209392**
**06.08.82 FR 8213804**
**20.09.82 FR 8215803**
**20.09.82 FR 8215804**
**27.10.82 FR 8218001**
**28.04.82 EP 82400770**

(43) Date de publication de la demande:
**29.06.83 Bulletin 83/26**

(45) Mention de la délivrance du brevet:
**10.05.89 Bulletin 89/19**

(84) Etats contractants désignés:
**AT BE CH DE FR GB IT LI LU NL SE**

(73) Titulaire: **D.R.O.P.I.C. (Société Civile), 10, Avenue Matignon, F-75008 Paris (FR)**

(72) Inventeur: **Choay, Jean, 21, rue Saint- Guillaume, F-75007 Paris (FR)**
Inventeur: **Jacquinet, Jean- Claude, 1, allée André Gide, F-45100 Orleans- La- Source (FR)**
Inventeur: **Petitou, Maurice, 27, rue du Javelot Appt. 201, F-75645 Paris Cedex 13 (FR)**
Inventeur: **Sinay, Pierre, 5, rue Jacques Monod, F-45100 Orleans (FR)**

(74) Mandataire: **Peaucelle, Chantal, S.C. Ernest Gutmann - Yves Plasseraud 67, boulevard Haussmann, F-75008 Paris (FR)**

(56) Documents cité:
**EP-A-0 064 012**

**CHEMICAL ABSTRACTS, vol. 76, no. 23, 5 juin 1972, page 539, no. 141273x, Columbus Ohio (USA); A.F. BOEHKOV et al.: "Synthesis of glucosiduronic acids by the orthoester method".**
**CHEMICAL ABSTRACTS, vol. 97, no. 25, 20 décembre 1982, page 896, no. 216607d, Columbus Ohio (USA); M.M. LITVAK et al:: "1,2-0-Cyanoethylidene derivatives of uronic acid. Synthesis and stereospecific glycosylation".**
**THE JOURNAL OF ORGANIC CHEMISTRY, vol. 41, no. 26, 24 décembre 1976, pages 4110-4112; G. MAGNUSSON: "Carbohydrate thio ortho esters. Synthesis and characterization".**
**HELVETICA CHIMICA ACTA, vol. 58, Fasc. 6, 24 septembre 1975, no. 204-205, pages 1847-1864; P.C. WYSS et al.: "205. Synthesis of heparin saccharides IV. Synthesis of disaccharides possessing the structure of a repeating unit of heparin".**
**TETRAHEDRON LETTERS, no. 41, 1975, pages 3527-3530, Pergamon Press, (GB); F.W. LICHTENTHALER et al.: "Nucleosides, XXVI. An alternate synthetic approach to gougerotin".**
**CARBOHYDRATE RESEARCH, vol. 94, 1981, pages C1-C4, Elsevier Scientific Publishing Company, Amsterdam (NL); V. BETANELI et al.:**

(56) Documents cité: (suite)

**"Stereospecific glycosylation by 1,2-0-cyanoethylidene derivatives of uronic acids".**

**THE JOURNAL OF ORGANIC CHEMISTRY, vol. 42, no. 5, 4 mars 1977, pages 913-914; G. MAGNUSSON: "Carbohydrate thio ortho esters. 3. Transformation to thioglycosides with dectivated Raney nickel".**
**CHEMICAL ABSTRACTS, vol. 87, no. 15, 10 octobre 1977, no. 118023j, Columbus Ohio (USA); G.MAGNUSSON: "Carbohydrate thio orthoesters: part II. Desulfuration of carbohydrate thio orthoesters with Raney nickel". & CARBOHYDR. RES. 1977, 56(1), 188-90.**
**TETRAHEDRON LETTERS, no. 30, 1972, pages 3055-3058, Pergamon Press, (GB); J. KISS et al.: "Syntheses of heparin saccharides stereospecific synthesis of derivatives of 2-amino-2-deoxy-4-C (alpha-D-glucopyranuronosyl) -D-glucose".**
**CARBOHYDRATE RESEARCH, vol. 93, no. 1, juin 1981, pages 144-147, Elsevier Scientific Publishing Company, Amsterdam (NL); P. KOVAC et al.: "Chemical synthesis of 2-0-(4-0-mehtyl-alpha-D-glucopyranosyluronic acid)-D-xylose".**
**CARBOHYDRATE RESEARCH, vol. 86, 1980, pages 177-183, Elsevier Scientific Publishing Company, Angewandte Chemie, 21(3),March 1982,p.155-172**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 082 793 B1

LIBER, STOCKHOLM 1989

**Description**

L'invention est relative à de nouveaux dérivés à structure acide uronique et à leur procédé de synthèse.

Elle concerne également les applications de ces dérivés, notamment, comme intermédiaires de synthèse osidique, substrats d'enzymes, haptènes et réactifs de laboratoires.

L'invention concerne plus particulièrement des dérivés des épimères acide D-glucuronique et acide L-iduronique.

On rappelle que ces épimères répondent respectivement aux formules suivantes: (I) et (II)

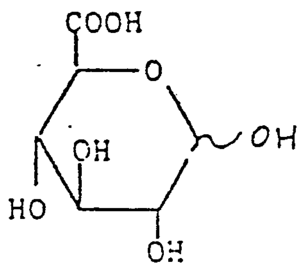
(I)

acide D-glucuronique

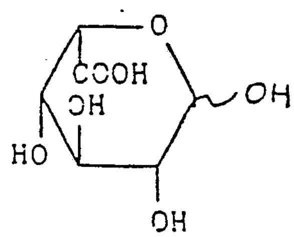
(II)

acide L-iduronique

Des motifs uroniques répondant à ces structures entrent dans la constitution des chaînes osidiques de composés biologiquement actifs de grande importance.

En particulier, de tels motifs sont présents dans divers mucopolysaccharides naturels tels que l'héparine, l'héparane sulfate, les chondroïtines et autres.

De nombreux travaux de la demanderesse ont porté sur la mise au point de moyens permettant d'obtenir par voie de synthèse des oligosaccharides correspondant à des fragments de mucopolysaccharides tels que ceux définis ci-dessus ou correspondant à des dérivés de cex fragments.

Les recherches effectuées à cet égard ont montré, notamment, en ce qui concerne les motifs à structure uronique, que pour conférer à une position donnée une réactivité suffisante permettant de l'impliquer, par exemple, dans une réaction de glycosylation, et/ou pour introduire, de manière spécifique, en des positions données, des substituants choisis, certains types de groupes protecteurs devaient être utilisés.

Or, en ce qui concerne plus particulièrement les structures acide iduronique, seuls quelques exemples de dérivés ont été décrits dans la littérature, à savoir, plus spécialement par KISS et al, Tetrahedron, 32, 1399 - 1402 (1976) et Srivastava et al dans Carb. Res. 60 (1978) 315 - 316.

De plus, la méthode d'oxydation sélective utilisée pour la synthèse de ces dérivés conduit à de faibles rendements et ne s'est pas avérée reproductible.

On conçoit donc qu'une telle méthode ne peut être utilisée pour fixer des motifs acide L-iduronique sur des composés de prix élevé.

Dans le C.A., volume 97, 1982, résumé 216607d, on décrit l'obtention de dérivés de 1,2-O-cyanoéthylidène d'acide uronique. Les dérivés de départ mis en oeuvre pour leur obtention comportent, contrairement à ceux visés par l'invention, trois substituants identiques sur les positions 2, 3 et 4. De plus dans les orthoesters, les positions 3 et 4 sont occupés par un groupe OAc (Ac désignant un groupe acyle).

Dans le C.A. volume 76 de 1972, résumé 141273x on décrit une synthèse d'acides glucosiduroniques dans laquelle on utilise comme produit de départ un dérivé dans lequel les positions 2, 3 et 4 sont substituées par un même groupe.

Dans le Journal of Organic Chemistry, volume 41 1976, pages 4110 - 4112, on rapporte des thio ortho esters dont les positions 3 et 4 sont occupées, comme dans le cas précédent, par des groupes OAc, alors que les positions 1 et 2 comportent un groupe ortho ester.

Dans Helvetica Chimica Acta, volume 58, 1975, pages 1847 à 1864 et dans Tetrahedron Letters n° 30, 1972 pages 3055 - 3058, les auteurs rapportent la synthèse de saccharides de type héparinique comprenant l'utilisation de dérivés glucuroniques substitués en position 2 par un groupe nitroso.

Dans Carbohydrate Research, 94, 1981, C1 - C4, on décrit des dérivés de 1,2-O-cyanoéthylidène d'acides uroniques dont les positions 3 et 4 sont occupées par des groupes OAc.

En fait, l'activation des dérivés uroniques de l'art antérieur, pour les impliquer dans une réaction de glycosylation n'a pas été envisagée par les auteurs ci-dessus.

Afin de disposer de motifs appropriés, en particulier pour les synthèses ci-dessus, les inventeurs ont été alors amenés à rechercher des moyens permettant d'accéder aisément aux structures acide D-glucuronique et acide L-iduronique en tant que telles et aux dérivés recherchés.

L'invention a donc pour but de fournir de nouveaux dérivés d'acide D-glucuronique et d'acide L-iduronique dotés d'une réactivité suffisante leur permettant d'être engagés dans des réactions de glycosylation en tant qu'agents glycosylants ou en tant qu'agents glycosylés (dans ce cas jouant le rôle d'aglycone selon la terminologie courante).

L'invention a également pour but de fournir de nouveaux dérivés permettant, grâce à la nature de leurs groupements protecteurs, l'introduction, de manière spécifique, en des positions données, de groupes substituants choisis.

Elle vise, en outre, à fournir de nouveaux dérivés dotés d'une réactivité suffisante leur permettant d'être engagés dans des réactions de glycosylation en tant qu'agents glycosylants ou en tant qu'agents glycosylés et permettant, grâce à la nature de leurs groupements protecteurs, l'introduction, de manière spécifique, en des positions données, de groupes substituants choisis.

Elle a également pour but de fournir des moyens permettant d'élaborer les structures des acides en question, en particulier celle de l'acide L-iduronique et d'accéder aisément aux dérivés réactifs ci-dessus.

L'invention vise également les applications de ces dérivés en synthèse osidique, notamment pour l'élaboration d'oligosaccharides correspondant, en particulier, à des fragments, ou des dérivés de fragments, des composés naturels ci-dessus, ou encore pour l'élaboration de glycosides utilisables comme réactifs biologiques, notamment comme substrats d'enzymes et/ou réactifs de laboratoires.

Elle vise également les applications de ces dérivés en tant que déterminants antigéniques où, conjugués à des agents de poids moléculaire élevé, ils peuvent induire, chez les animaux, la formation d'anticorps dirigés contre eux-mêmes.

Elle vise également les applications de ces conjugués en vue de la fabrication d'immunoabsorbants utilisables, notamment, pour la purification des anticorps ci-dessus ou de tout autre substance ayant une affinité à leur égard.

Les dérivés à structure acide uronique de l'invention sont caractérisés en ce qu'ils répondent à la formule (III):

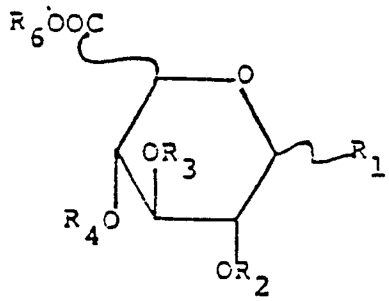

(III)

dans laquelle:

$R_1$ représente un groupe réactif, choisi parmi

. un halogénure, de préférence un chlorure ou un bromure,

. un groupe -O-imidoyle, ou

. conjointement avec le substituant voisin $-OR_2$ un groupe orthoester,

ou encore

. un groupe -O-alcényle, de préférence -O-allyle,

ou

. un groupe -OH libre ou bloqué par un groupement protecteur choisi parmi les radicaux aliphatiques ou aromatiques, en particulier, dans le groupe comprenant un radical alcoyle de 1 à 4 atomes de carbone, tel que méthyle, un radical alcoyle substitué, tel que benzyle, un radical acyle tel qu'acétyle, benzoyle ou chloroacétyle, ou un radical alcényle de 1 à 4 atomes de carbone, tel qu'allyle ou vinyle, étant entendu que lorsque $R_1$ représente un groupe méthoxy, $OR_4$ est différent d'un tel groupe,

$R_2$, $R_3$ et $R_4$ qui, dans le cas de dérivés glucuroniques ne peuvent être tous trois identiques, $R_3$ et $R_4$ étant, en outre, différents d'un groupe acétyle, lorsque $R_1$ et $R_2$ forment conjointement un orthoester, $R_2$ et $R_3$ étant différents d'un groupe benzyle, lorsque $R_1$ représente un atome de chlore et $R_4$ représente un groupe méthyle, ou lorsque $R_1$ représente un groupe -O-alcoyle et $R_4$ un atome d'hydrogène,

représentent

. un groupe alcényle, de préférence un groupe allyle,

. un atome d'hydrogène ou

. un groupe OH bloqué par un groupement protecteur tel que défini ci-dessus pour $R_1$,

$R_6$ représente un atome d'hydrogène ou un groupe protecteur tel que défini ci-dessus, ou les sels de ces dérivés, à l'exclusion des dérivés d'acide iduronique dans lesquels $R_1$, $R_2$ et $R_3$ représentent tous trois un groupe benzyle et $R_6$ représente un groupe méthyle.

On remarquera que les dérivés à structure iduronique de l'invention comprennent des dérivés semi-ouverts et des dérivés ouverts.

On appelle dérivé semi-ouvert un dérivé activé ou potentiellement activable sur une position.

Un dérivé semi-ouvert à droite est un dérivé capable d'être engagé par sa position 1 dans une réaction avec un autre composé.

Un dérivé semi-ouvert à gauche comporte une seule fonction libre, plus spécialement un seul groupe -OH libre susceptible d'être engagé dans une réaction.

Un dérivé ouvert est donc un dérivé semi-ouvert à droite et à gauche et peut donc être utilisé comme agent glycosylant ou glycosylé selon les cas.

Un tel dérivé renferme alors un groupe protecteur temporaire, c'est-à-dire éliminable indépendamment des autres groupes protecteurs présents, en recréant un alcool.

Des dérivés de l'invention à structure D-glucuronique sont des dérivés ouverts.

Selon une autre disposition avantageuse de l'invention, les groupes protecteurs $R_2$ et $R_3$, et le cas échéant $R_4$ représentent des groupes protecteurs différents, et éliminables séquentiellement sans altération de la structure uronique et de la fonction carboxyle.

Cette disposition présente l'intérêt, au cours de réactions ultérieures, de pouvoir introduire à la place de tous ces groupes protecteurs des substituants différents entre eux ou, en variante, d'introduire seulement un ou plusieurs substituants et de libérer les groupes -OH des positions restantes.

Dans des dérivés préférés de l'invention, le groupe réactif occupe la position 1.

Une famille préférée de dérivés de l'invention correspond aux dérivés d'acide D-glucuronique de formule (IV):

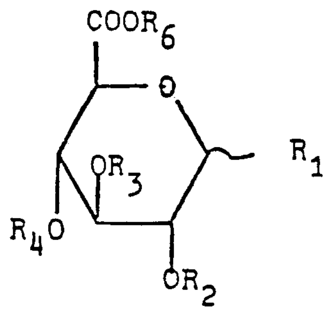

(IV)

dans laquelle:

$R_1$ représente un groupe réactif choisi parmi

. un halogénure, de préférence un chlorure ou un bromure,

. un groupe -O-imidoyle ou,

. conjointement avec $OR_2$, un groupe orthoester,

. un radical -O-alcoyle-substitué ou encore un radical alcényle, de préférence -O-allyle,

. un groupe -OH,

$R_2$ à $R_4$ ne pouvant être tous trois identiques, représentent un groupe protecteur choisi parmi un radical alcoyle de 1 à 3 atomes de carbone, de préférence méthyle, alcoyle substitué, de préférence benzyle, acyle, de préférence acétyle, benzoyle ou chloroacétyle, ou alcényle, de préférence allyle ou vinyle,

$R_6$ représente un radical aliphatique ou aromatique, en particulier un radical alcoyle de 1 à 4 atomes de carbone, ou alcoyle substitué, tel que benzyle et les sels de ces dérivés.

Dans un groupe préféré de dérivés d'acide uronique, au moins l'un des groupes $R_2$ ou $R_3$ représente un groupe acyle.

Dans un autre groupe préféré de dérivés d'acide uronique, $R_4$ représente un groupe temporaire formant un ester ou un éther avec le groupe -OH qu'il protège. Ce groupe temporaire est avantageusement choisi parmi les radicaux du type monochloroacétyle, acétyle, benzyle, p-méthoxybenzyle, allyle ou vinyle.

Des produits préférés de ce groupe renferment un radical $R_1$ représentant un groupe allyle ou vinyle. De tels produits présentent l'avantage de porter en positions 1 et 4 des substituants éliminables sélectivement sans par ailleurs altérer les groupements en positions 2, 3 et 6.

Dans un autre groupe préféré $R_1$ représente un groupe -OH ou bien $R_4$ représente un groupe -OH et $R_1$ un groupe alcényle tel que O-allyle.

Une autre famille préférée de dérivés de l'invention correspond aux dérivés de l'acide L-iduronique de formule (V):

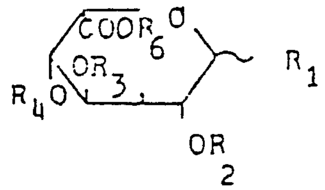

(V)

dans laquelle les substituants présentent les significations générales données ci-dessus en rapport avec la formule III.

Dans un groupe préféré de dérivés d'acide L-iduronique, $R_1$ et $-OR_2$ représentent un orthoester.

Dans un autre groupe préféré, $R_1$ est choisi parmi:

. un halogénure,
. un radical alcoyle
. ou un radical alcoxyle.

Des produits préférés de ces groupes renferment avantageusement un groupe temporaire en position 4.

D'autres produits particulièrement préférés de ces groupes renferment en outre des substituants $R_2$ et $R_3$ différents, ce qui permet au cours d'opérations ultérieures de soumettre ces groupes à des traitements différents et par là d'introduire des groupes de substitution différents sur le motif.

Dans le cas où, par exemple, $R_2$ représente un groupe acyle tel qu'un groupe acétyle et $R_3$ un groupe benzyle, il est possible d'introduire en position 2 un groupe sulfate à la place du groupe acétyle, puis de libérer en position 3 le radical -OH bloqué par le groupe benzyle.

On dispose ainsi selon une voie aisée d'un motif constitutif de chaînes d'héparine ou d'héparane-sulfate.

D'autres produits préférés renferment des substituants $R_2$ et $R_3$ différents l'un de l'autre.

L'invention vise également un procédé de synthèse de ces dérivés.

Selon ce procédé, on introduit sur un monosaccharide à structure uronique des groupes protecteurs compatibles entre eux, autorisant le traitement sélectif d'une position donnée sur laquelle on désire introduire un groupe réactif ou, le cas échéant, un groupe protecteur donné.

On appellera groupe protecteur intermédiaire un groupe utilisé durant le processus de synthèse puis éliminé pour la mise en place d'un groupe réactif ou d'un groupe protecteur final, c'est-à-dire correspondant au groupe protecteur souhaité sur le monosaccharide de l'invention.

On peut introduire, dès le départ, un ou plusieurs groupes protecteurs finaux.

En variante, l'introduction d'un ou plusieurs groupements protecteurs intermédiaires permet, dans un premier temps, de traiter une position donnée, puis lorsque le groupement désiré a été introduit dans cette position, de traiter indépendamment le ou les positions occupées par ce ou ces groupes intermédiaires et, le cas échéant, successivement, aux fins d'introduction de groupes protecteurs finaux différents.

Pour préparer, par exemple, un monosaccharide comportant un groupe vinyle, on élabore avantageusement un dérivé allyle dans lequel tous les groupes -OH des fonctions alcool sont bloqués par des groupements protecteurs finaux ou, le cas échéant, intermédiaires. On soumet ce monosaccharide allyle à l'action d'un agent d'alcoylation afin de bloquer le groupe -OH de la fonction carboxyle. Le monosaccharide à groupe allyle ainsi protégé est alors soumis, par exemple, à l'action d'un complexe de rhodium en présence de diazabicyclo-octane, ce qui conduit à l'éther vinylique correspondant.

On remarquera que dans le cas où un groupe temporaire a été introduit en une position donnée, il est ensuite possible d'éliminer ce groupe temporaire et de recréer en cette position un alcool utilisable pour une réaction de glycosylation tandis que le groupe vinyle peut être, à son tour, soumis à une ou plusieurs réactions pour allonger la chaîne.

A partir d'un dérivé 1-O-vinyle protégé, il est possible, par exemple, d'élaborer un groupe réactif tel qu'un imidate. A cet effet, on soumet avantageusement le dérivé vinyle à l'action de dérivés mercuriques tels que de l'oxyde mercurique et du chlorure mercurique ce qui permet de recréer un alcool que l'on soumet alors à l'action d'un agent d'imidoylation tel que le trichloroacétonitrile.

Il est également possible d'obtenir un imidate à partir d'un dérivé 1-O-hydroxylé obtenu par hydrolyse acide d'un glycoside et en particulier d'un méthylglycoside.

De manière classique, les monosaccharides à structure uronique halogénés sont avantageusement obtenus par action d'un agent d'halogénation sur un groupe acyle, lui-même obtenu, le cas échéant, par acylation d'un alcool. On peut également les obtenir par action du réactif de Vilsmeier sur un dérivé hydroxylé en position 1.

On peut également préparer un monosaccharide comportant un groupe orthoester en opérant avantageusement à partir du dérivé halogéné correspondant. Selon un mode préféré d'obtention, on soumet le dérivé halogéné, en présence d'un accepteur de protons tel que de la symcollidine, à l'action d'un alcool.

Selon une disposition avantageuse de l'invention, les monosaccharides à structure L-iduronique sont préparés à partir du 3-O-benzyl-iduronate de méthyle lui-même élaboré à partir d'un dérivé de type D-glucofuranoside, selon les étapes suivantes:

- on traite un dérivé d'α-D-glucofuranoside de formule VI:

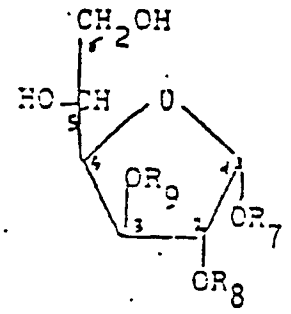

(VI)

dans laquelle les groupes $R_7$ à $R_9$ peuvent être identiques ou différents les une des autres et représentent des groupes de blocage de radicaux hydroxyle tels que définis ci-dessus, $R_7$ et $R_8$ formant, de préférence, un groupe isopropylidène et $R_9$ un groupe benzyle,

- on effectue la conversion de la structure D-gluco en position 5 en une structure L-ido,
- on effectue le passage de la structure alcool primaire en position 6 en une structure acide carboxylique,
- on procède au réarrangement de la forme furanose en la forme pyranose.

Le traitement sélectif des groupes -OH de la chaîne α-gluco et l'utilisation de la chaîne formée pour passer du cycle glucofuranoside au cycle idofuranoside puis idopyranoside sont effectués selon un procédé qui comprend:

1 - le blocage sélectif du groupe -OH en position 6, en utilisant du chlorure de trityle,
2 - le blocage sélectif du groupe -OH de -CHOH en position 5, avantageusement par un groupe protecteur temporaire tel que benzoyle,
3 - le déblocage sélectif du groupe -OH en position 6 afin d'obtenir un groupe $-CH_2OH$.

Le déblocage du groupe hydroxyle est avantageusement réalisé avec de l'acide paratoluène sulfonique, ou encore, avec une solution de trifluorure de bore dans le méthanol.

4 - l'oxydation du groupe $-CH_2OH$ en un groupe -COOH, en utilisant un agent d'oxydation tel que de l'oxyde chromique,
5 et 6 - le déblocage du groupe -OH en position 5 par traitement par de la soude et la transformation, si souhaitée, du groupe -COOH en position 6 en un groupe -COOMe, en utilisant un agent d'estérification approprié, tel que le diazométhane.
7 - l'introduction en position 5 d'un groupe réactif tel qu'un tosylate, un mésylate, un groupe triflyle,
8 - l'inversion de configuration du carbone en position 5, à l'aide d'un agent nucléophile,
9 - l'élimination du groupe fonctionnel en position 5, à l'aide de méthanol, suivie avantageusement d'une chromatographie aux fins de purification.
10 - le réarrangement du cycle pour passer d'un cycle idofuranosique à un cycle idopyranosique, en opérant en milieu acide.

On opère avec avantage en milieu acide, en particulier en présence d'acide trifluoroacétique. Un mélange acide trifluoroacétique/eau de l'ordre de 9/1 s'avère spécialement approprié. L'invention vise également en tant que produits intermédiaires, les furanosides à structure D-gluco et L-ido et les idopyranosides obtenus à l'issue des étapes 2 à 8 ci-dessus.

En ce qui concerne plus particulièrement le composé de structure acide L-iduronique de formule V obtenu dans lequel $R_1$ représente un groupe alcoyle, en particulier méthyle, on notera qu'il peut être saponifié puis soumis à une reaction d'hydrogénation catalytique aux fins d'obtention d'un motif acide L-iduronique.

Ce composé peut être encore acylé, transformé en halogénure puis en ortho-ester. Le groupement protecteur en position 4 peut être éliminé permettant ainsi l'introduction d'un autre groupe protecteur temporaire en position 4.

Moyennant un choix convenable du groupement protecteur en position 4, et selon la nature de l'orthoester, il est possible, à partir de ces composés de structure V, d'effectuer une réaction de glycosylation suivie d'une élimination sélective du groupement protecteur en position 4; cette position peut ainsi être engagé dans une autre réaction de glycosylation.

Enfin, l'ester ou le groupement acyle formé en position 2 au cours de la première réaction de glycosylation peut être éliminé, régénérant ainsi une fonction -OH que l'on peut sulfater, si souhaité.

Selon un autre mode de réalisation, on prépare un monosaccharide du L-idose possédant, en positions 1, 2, 3 et 4 des groupements protecteurs souhaités puis on oxyde chimiquement, sans altérer les groupements protecteurs ci-dessus, la seule fonction alcool primaire en position 6 restée libre, puis on oxyde en groupe en acide carboxylique.

Les monosaccharides à structure D-glucuronique sont plus spécialement élaborés à partir de dérivés de glucose avec mise en jeu des techniques classiques pour l'introduction des groupes souhaités.

On remarquera, dans cette synthèse, que le passage de la structure D-gluco à la structure L-ido au stade acide méthylester, passage réalisé grâce à l'utilisation d'un groupe triflate et de trifluoroacétate desodium, permet d'effectuer l'inversion souhaitée au stade méthyl ester.

Ces conditions sont les seules mises au point à ce jour pour permettre cette réaction.

Les moyens mis en oeuvre selon l'invention pour obtenir les dérivés définis ci-dessus, en particulier les dérivés à structure iduronique, présentent l'avantage, par rapport aux techniques connues jusqu'à présent, de permettre une mise en oeuvre aisée et reproductible.

Grâce à la grande souplesse de ces moyens, il est possible d'accéder à des dérivés à structure uronique capables de donner lieu à divers types d'enchaînements selon une stéréochimie préférée grâce à leur réactivité. En outre, selon le type des groupes protecteurs choisis, il est possible après engagement dans un type donné de réaction d'introduire sélectivement à leur place un groupe de substitution donné et/ou de libérer des groupes -OH.

Il est ainsi possible de disposer d'oligosaccharides comportant des motifs uroniques, substitués de manière spécifique tels que rencontrés par exemple dans les chaînes de molécules biologiquement actives ci-dessus.

On considère plus particulièrement à cet égard l'intérêt de pouvoir accéder aisément à un dérivé de l'acide L-iduronique -2-O-sulfate, substrat de l'enzyme L-iduronate sulfatase.

De même, la sulfatation mise au point par la Demanderesse de l'acide D-glucuronique permet d'envisager la synthèse de dérivés de cet acide sulfatés pouvant être utilisés comme substrats d'enzymes.

De telles synthèses ne sont possibles que grâce au jeu de groupements protecteurs décrits ci-dessus.

L'invention vise également l'application des dérivés d'acide uronique de l'invention à la synthèse de glycosides présentant un intérêt biologique tels que les substrats d'enzymes de la famille des glycosidases.

De tels glycosides résultent par exemple de la condensation des dérivés de l'invention avec du para-nitrophénol ou de la méthylumbelliférone ou de leurs dérivés. A cet effet, on peut mettre en oeuvre une réaction du type Koenigs-Knorr, bien connue de l'homme de l'art, dans laquelle on met en présence un halogénure de sucre, un aglycone (avec lequel on peut établir la liaison) et un agent capteur de l'acide halohydrique formé.

On notera particulièrement, en ce qui concerne l'acide iduronique, que la liaison glycosidique est établie alors que la structure acide uronique est déjà élaborée. Cette disposition permet la glycosylation de produits ou substances telles que des protéines ou des supports solides qui ne pourraient pas supporter les etapes de synthèse du procédé de l'art antérieur évoqué ci-dessus dans lequel la liaison glycosidique est établie avant l'élaboration de la structure acide iduronique.

Les substrats d'enzymes élaborés à partir de monosaccharides de l'invention permettent avantageusement d'étudier la spécificité de diverses enzymes, en particulier d'enzymes dégradant l'héparine, notamment de la L-iduronidase, la D-glucuronidase et la L-iduronate sulfatase.

Les glycosides obtenus par fixation des monosaccharides de l'invention, par exemple sur la méthylumbelliférone constituent des composés précieux comme substrats utilisables en fluorescence.

Dans d'autres applications, les monosaccharides de l'invention sont utilisés pour l'élaboration de substrats radioactifs. Ces substrats peuvent être obtenus en mettant en oeuvre un glycoside de départ radioactif ou en réduisant les groupes nitrophénylglycosides évoqués ci-dessus pour les études d'enzymes, en groupé amine, soumis ensuite, par exemple, à une réaction d'acétylation avec de l'anhydride acétique tritié selon les techniques classiques.

On notera également dans cette application, l'intérêt de pouvoir disposer d'un dérivé possédant déjà la structure acide uronique. On conçoit, en effet, l'importance de la réduction au minimum du nombre des étapés auxquelles on doit soumettre le composé radio-actif pour l'élaboration d'un glycoside donné compte-tenu de son prix élevé.

Selon un autre aspect de l'invention de grand intérêt, les monosaccharides renfermant un groupe fonctionnalisable sur le carbone anomère ouvrent la voie à l'introduction de chaînes capables de jouer le rôle de bras ancreurs utilisables dans diverses applications biologiques.

Selon encore un autre aspect, certains glycosides peuvent être transformés en bras ancreurs et permettent la fixation à une protéine, constituant ainsi des antigènes artificiels. A cet effet, on soumet, par exemple, un glycoside tel que le dérivé de p-nitrophényl glycoside, évoqué ci-dessus, à une réaction de réduction, ce qui conduit au dérivé p-amino phényl glycoside correspondant et ce dernier, par réaction classique de couplage diazoïque est alors engagé dans en liaison impliquant par ailleurs les résidus aromatiques et, en particulier tyrosine des protéines.

De tels antigènes sont utilisés chez l'animal pour induire la formation d'anticorps qui peuvent être purifiés et utilisés comme réactifs immunologiques.

A partir d'un glycoside tel qu'évoqué ci-dessus ou d'un substituant fonctionalisable d'un dérivé d'acide uronique, il est possible d'élaborer, des bras ancreurs variés comportant notamment (1) un radical alcoyle de 2 à 10 atomes de carbone, comportant en bout de chaîne un groupe -OH, éventuellement protégé ou engagé dans un groupe fonctionnel (2) un radical alcényle comprenant de 2 à 10 atomes de carbone.

Des chaînes de type (1) correspondent avantageusement à des radicaux, alcoylène-glycol α,β-dihydroxypropyle, ou β-hydroxyéthyle, γ-hydroxypropyle et sont utilisables pour la constitution de réactifs biologiques.

Les chaînes (1) et (2) ci-dessus renferment avantageusement en outre un ou plusieurs groupements éther et/ou amine intercalaire et/ou un groupe fonctionnel terminal renfermant de l'azote tel qu'une fonction amine ou un groupe terminal éther, carboxyle ou aldéhyde.

Des chaînes à terminaison amino, particulièrement préférées sont du type alcoxy-alcoylamine dans lequel les groupes alcoxy et alcoyle renferment ensemble de 3 à 5 atomes de carbone, de préférence de 4 à 10, avantageusement de l'ordre de 10.

De telles chaînes sont avantageusement obtenues à partir des dérivés allyle de l'invention selon le procédé décrit dans la demande de brevet FR-7 810 558 au nom de l'AGENCE NATIONALE DE VALORISATION DE LA RECHERCHE.

La réactivité du groupe allyle permet, en effet, l'articulation d'un grand nombre de réactions, et par là, l'obtention de chaînes de longueur et de nature modulables, à caractère hydrophile précieux pour les applications biologiques.

Pour l'allongement de la chaîne, on a recours aux reactions classiques de la synthèse organique, qui seront aisément mises en oeuvre par l'homme de l'art en fonction de la chaîne souhaitée.

Il est, par exemple, possible d'obtenir une chaîne terminée par un groupement fonctionnel alcool, en particulier par ozonolyse de la double liaison du groupe allyle, puis de recourir à une tosylation et, enfin, de faire réagir le dérivé tosylé avec un azide.

L'azide obtenu présente un double intérêt. Il s'agit, d'une part, d'un composé stable. D'autre part, par réduction, selon les techniques classiques, il permet d'obtenir aisément des chaînes de substitution à groupe amino terminal particulièrement précieuses pour la constitution des immunoadsorbants de l'invention.

Les monosaccharides à bras ancreurs tels qu'évoqués ci-dessus constituent de précieux réactifs biologiques utilisables, en particulier, sous forme immobilisée sur des supports solides afin de constituer des immunoabsorbants.

Ces immunoabsorbants peuvent particulièrement servir à la purification des anticorps obtenus ci-dessus, ou encore à la purification de toute substance ayant pour eux de l'affinité. Ils peuvent également être utilisés dans des techniques de dosage mettant à profit cette affinité.

Des supports solides appropriés sont constitués par exemple par des polysaccharides, tels que la cellulose, l'agarose ou l'agarose réticulé (notamment celui commercialisé sous la marque SEPHAROSE par PHARMACIA), ou des polymères mixtes renfermant outre les polysaccharides ci-dessus des polymères tels que des polyacrylamides (notamment ceux commercialisés sous les marques ULTROGEL et MAGNOGEL par IBF), ou encore des silices greffées portant des groupements fonctionnels appropriés.

Des monosaccharides de l'invention peuvent également constituer des haptènes conjugables à des supports solubles, tels que la BSA (albumine de sérum bovin) ou la poly-lysine destinés à la préparation d'anticorps polyclônaux ou monoclônaux.

Outre leur utilisation comme déterminants antigéniques, les produits de l'invention sont précieux comme produits de référence pour des études structurales.

D'autres caractéristiques et avantages de l'invention apparaîtront dans les exemples qui suivent et en se référant aux figures 1 à 16. Les références numériques utilisées dans ces figures pour désigner les dérivés sont également reprises dans les exemples. Dans ces figures, les symboles utilisés présentent les significations suivantes:

A représente un groupe allyle, Ø un groupe $-C_6H_5$, Bn un groupe benzyle, Tr un groupe trityle, $R_6$ un groupe -OAc ou $-OB_z$, avec Ac représentant un groupe acétyle et Bz un groupe benzoyle, Me un groupe méthyle P un groupe vinyle, MCA un groupe monochloroacétyle, Tf un groupe triflyle, t-Bu un groupe tertio-butyle et $SnBu_3$ le radical tributyl stanyle.

Les formules illustrant les composés des exemples 1 à 6 sont rapportées sur les figures 1 et 2.

**Exemple 1**

Préparation de l'acide (allyl-4-O-acétyl-2,3-di-O-benzyl-α-D-glucopyranoside) uronique (composé 8a)

On effectue cette synthèse à partir du glucose, selon les étapes a) à g) suivantes:

a) Préparation de l'allyl-α-D-glucopyranoside (composé 1)

Une solution d'acide chlorhydrique gazeux (18 g) dans de l'alcool allylique (600 ml) est chauffée à 70°C. On ajoute alors du glucose anhydre (300 g) et on maintient à cette température pendant 3 heures.

La réaction peut être suivie en chromatographie sur couche mince (c.c.m.) dans le solvant méthanol/chloroforme (1/4, v/v). La solution brune obtenue après 3 heures est concentrée à sec, sous vide, neutralisée par une solution concentrée d'ammoniaque (50 ml) puis concentrée à nouveau à sec. Au résidu obtenu, on ajoute del'acétone (500 ml), on porte à ébullition et on maintient ainsi jusqu'à dissolution totale. Après refroidissement, le liquide est décanté. Le résidu est denouveau soumis au même traitement jusqu'à ce que l'analyse en c.c.m. de l'extrait montre un épuisement du résidu en dérivé 1 ou bien une trop forte

contamination de l'extrait par des impuretés.

Une partie de la première fraction extraite (12 g) est chromatographiée sur silice. On récupère le dérivé 1 qui peut être cristallisé dans un mélange acétone/éther (6,5 g; pf 95 - 99°C). Le reste du produit peut être purifié selon le même processus.

b) Préparation de l'allyl 4,6-O-benzylidène-α-D-glucopyranoside (composé 2)

Le composé 1 (37 g) est dissous dans du diméthylformamide (200 ml). Du diméthoxytoluène (41 g) est alors ajouté suivi d'acide paratoluène sulfonique hydraté (130 mg). Après 2 heures de chauffage (bain-marie) sous vide et reflux, la réaction est terminée (c.c.m. méthanol/chloroforme, 2/25, v/v). Le solvant est évaporé. Le sirop est dissous dans du méthanol (le minimum), cette solution est versée goutte à goutte dans une solution aqueuse de bicarbonate de sodium (6,3 g dans 320 ml d'eau). Le précipité obtenu est recristallisé dans l'éthanol (21 g; p.f. 120 - 121°C). Les eaux-mères livrent encore du produit 2. Rendement total (37 g; 71,4 %).

c) Préparation de l'allyl 2,3-di-O-benzyl-4,6-O-benzylidène-α-D-glucopyranoside (composé 3)

Le composé 2 (45 g) est dissous dans du DMF anhydre (500 ml). De l'hydrure de sodium (28 g d'une dispersion à 50 % dans l'huile) est ajouté.

Après 30 minutes, le mélange est refroidi à 0°C et on ajoute alors, goutte à goutte, du bromure de benzyle (52 ml). La réaction est suivie en c.c.m. (éther/hexane, 1/1, v/v). On ajoute ensuite lentement du méthanol (150 ml), évapore à sec et reprend par du chloroforme. La phase chloroformique est lavée avec de l'eau, séchée sur sulfate de sodium. Après évaporation du solvant, le résidu est cristallisé dans un mélange éther/hedane (36,5 g: PF 83 - 84°C).

Ce produit est légèrement contaminé par une impureté migrant plus haut en c.c.m. (éther/hexane; 1/1; v/v).

d) Préparation de l'allyl 2,3-di-O-benzyl-α-D-glucopyranoside (composé 4)

A une solution du composé 3 (56 g) dans le méthanol (1 l), on ajoute de l'eau (450 ml) puis de l'acide paratoluène sulfonique hydraté (17 g).

Après 2 heures à 80°C, on laisse refroidir le mélange, on évapore le solvant et on reprend le résidu par du chloroforme (1 l). La solution chloroformique est lavée avec de l'eau jusqu'à pH neutre, puis séchée sur sulfate de sodium. On obtient ainsi un sirop jaune pâle (48 g) qui est engagé dans l'étape suivante (synthèse du compose 5).

e) Préparation de l'allyl 2,3-di-O-benzyl-6-O-trityl-α-D-glucopyranoside (compose 5) et de son analogue 4-O-acétylé (composé 6a)

Le dérivé 4 obtenu (48 g) est dissous dans de la pyridin (250 ml) et du chlorure de trityle (38,5 g) est ajouté. Après 1 heure à 100°C, la réaction est terminée (c.c.m. éther/hexane, 1/1, v/v). A la solution précédente, on ajoute de l'anhydride acétique (200 ml). Après une nuit, la réaction est complète (c.c.m.; éther/hexane, 1/2, v/v). On évapore à sec, reprend le résidu par du chloroforme (500 ml), lave la phase chloroformique avec une solution de sulfate acide de potassium à 10 % avec de l'eau et sèche sur sulfate de sodium.

Le chloroforme est évaporé. On obtient ainsi le composé 6a qui est engagé tel quel dans la réaction de préparation du composé 7a.

f) Préparation de l'allyl 4-O-acétyl-2,3-di-O-benzyl-α-D-glucopyranoside (composé 7a)

Le dérivé 6a obtenu est dissous dans du chloroforme (500 ml). A cette solution, refroidie à 0°C, on ajoute goutte à goutte sous agitation, une solution de trifluorure de bore dans le méthanol (20 %, 120 ml). La réaction est suivie c.c.m. (toluène/acétone, 10/2, v/v).

Le mélange réactionnel est transvasé dans une ampoule à décanter. La phase chloroformiqué est lavée par de l'eau (2 fois 100 ml) par une solution saturée de bicarbonate de sodium, puis avec de l'eau jusqu'à pH neutre. Après séchage, et évaporation, le résidu obtenu est introduit sur une colonne de gel de silice (500 g) équilibrée dans le toluène.

Après élution de la plupart des impuretés par le toluène pur, le produit est élué par un mélange toluène/acétone (10/2, v/v). On obtient ainsi 48 g du composé 7a qui sera engagé directement dans la synthèse du composé 8a. Une partie du composé 13a a été obtenue pure: $[\alpha]^{20}_D = +11°$, (chloroforme). Ses spectres IR et RMN, de même que l'analyse élémentaire, confirment la structure.

g) Préparation de l'acide (allyl-4-O-acétyl-2,3-di-O-benzyl-α-D-glucopyranosidé) uronique (composé 8a)

Une solution du composé 7a (48 g) dans l'acétone (800 ml) est refroidie à -5°C. On ajoute ensuite goutte à goutte, une solution de trioxyde de chrome (30 g) dans l'acide sulfurique (3,5 M; 125 ml). On laisse le mélange revenir à la température ambiante. La réaction est contrôlée en c.c.m. (méthanol/chloroforme, 1/10, v/v). A la fin de la réaction, le mélange réactionnel est versé dans de l'eau (500 ml). Le produit est extrait par le

chloroforme (3 fois 250 ml). La phase chloroformique est lavée à l'eau jusqu'à pH neutre, séchée sur sulfate de sodium et concentrée à sec.

Le sirop obtenu (83 g) est utilisé tel quel pour la préparation du composé 9a.

**Exemple 2**

Préparation de l'acide (allyl 4-O-benzoyl-2,3-di-O-benzyl-α-D-glucopyranoside) uronique (composé 8b) et du méthyl ester correspondant (composé 9b)

Dans une première étape, on prépare tout d'abord l'allyl 4-O-benzoyl-2,3-di-O-benzyl-6-O-trityl-α-D-glucopyranoside (composé 6b) et l'allyl-4-O-benzoyl-2,3-di-O-benzyl-α-D-glucopyranoside (composé 7b)

1) Préparation des composés 6b et 7b

Le composé 6b est obtenu comme il est décrit pour 6a: à la solution pyridinique du composé 5, on ajoute alors du chlorure de benzoyle (1,5 équivalents) et la réaction est suivie en c.c.m. (acétate d'éthyle/benzène, 1/20, v/v).

L'excès de chlorure de benzoyle est détruit par addition d'un excès de méthanol. Après évaporation à sec, le résidu, repris par du chloroforme, est lavé avec une solution de $KHSO_4$ à 10 %, avec de l'eau, séché et concentré à sec. Le sirop obtenu est engagé tel quel dans la synthèse du composé 7b. Ce sirop (105 g, obtenu à partir de 30 g de composé 3) est dissous dans du chloroforme (300 ml). De l'acide paratoluène sulfonique (76 g de monohydrate dans 100 ml de méthanol) est ajouté. Après une nuit, la réaction est terminée (c.c.m., acétate d'éthyle/chloroforme, 1/20, v/v). La phase chloroformique est lavée avec de l'eau jusqu'à pH neutre, sechée et concentrée à sec. Le sirop obtenu (98 g) est chromatographié sur une colonne de gel de silice (1,2 kg), éluée avec du chloroforme (0,6 l) puis avec un mélange acétate d'éthyle/chloroforme (1/20, v/v). On obtient ainsi le dérivé 7b pur (30 g) qu'on engage tel quel dans l'étape de préparation du composé 8b.

**Exemple 3**

Préparation de l'(allyl 4-O-acétyl-2,3-di-O-benzyl-α-D-glucopyranoside) uronate de méthyle (composé 9a)

Le sirop obtenu à l'étape de préparation du composé 8a est dissous dans de l'éther (300 ml). Une solution éthérée de diazométhane est alors ajoutée jusqu'à disparition du composé 8a (c.c.m. éther/hexane, 1/1, v/v). Après acidification par l'acide acétique, les solvants sont évaporés.

Le résidu obtenu (53 g) est dissous dans l'éthanol à chaud. Le dérivé 9a cristallise au refroidissement. Après recristallisation, on obtient ce composé 9a pur (18,4 g) - pf 85 - 86° C - $[\alpha]^{20}_D = +12°$ (1,2 chloroforme).

Ce produit est caractérisé par ses spectres IR, RMN et par son analyse élémentaire.

A partir du filtrat de cristallisation, on obtient encore 7,6 g du composé 9a.

Le rendement global en 9a à partir du composé 2 est de 38 %.

**Exemple 4**

Préparation du (prop-1'-ényl 4-O-acétyl-2,3-di-O-benzyl-α-D-glucopyranoside) uronate de méthyle (composé 10a)

Le dérivé 9a (4 g) est dissous dans un mélange d'éthanol (119 ml) de benzène (51 ml) et d'eau (17 ml). On ajoute ensuite du diazabicyclo octane (170 mg) et on porte à reflux. On ajoute à la solution bouillante du chlorure de Tris (triphénylphosphine)-rhodium (I) (550 mg). L'ébullition est maintenue pendant 4 heures (c.c.m., éther/hexane, 1/1, v/v).

A la fin de la réaction, la solution est filtrée et les solvants sont éliminés. Le résidu est chromatographié sur gel de silice (150 g) dans un mélange acétate d'éthyle/chloroforme (1,50, v/v). On obtient le composé 10a (3,25 g; 81 %) qui cristallise dans l'éthanol. $[\alpha]^{20}_D = +12°$ (1, chloroforme). PF 90° C. La structure est confirmée par l'analyse élémentaire et les spectres RMN et IR.

**Exemple 5**

Préparation de l'acide (allyl 2,3-di-O-benzyl-α-D-glucopyranoside) uronique et de l'(allyl 2,3-di-O-benzyl-α-D-glucopyranoside) uronate de méthyle (composés 11 et 12)

Le composé 8b (1,9 g) est dissous dans du méthanol (40 ml). On ajoute alors de la soude (5N) en quantité suffisante pour avoir une concentration de 1 M en soude. La réaction est suivie en c.c.m. (méthanol/chloroforme, 1/4, v/v). Quand elle est terminée, on ajoute de l'eau (100 ml). On lave avec de l'éther, on acidifie et on extrait le produit à l'éther. La phase étherée acide est lavée avec de l'eau jusqu'à pH neutre. Le dérivé 11 n'est pas isolé. Il est méthylé par une solution éthérée de diazométhane, donnant ainsi le composé 12 (900 mg; 56 %) qui est alors purifié sur une colonne de gel de silice (éther/hexane, 1/1, v/v). $[\alpha]^{20}_D = +35,2°$ (1, 3, chloroforme). Ses spectres IR et RMN et son analyse élémentaire confirment sa structure.

De la même manière, le dérivé 11 et donc 12 peuvent être obtenus à partir de 9a à 9b.

**Exemple 6**

Préparation du (prop-1'-ényl 2,3-di-O-benzyl-α-D-glucopyranoside) uronate de méthyle (composé 13)

1° A partir du composé 12 - Le dérivé 12 est traité par le complexe au rhodium comme décrit pour 9a. Le composé 13 est obtenu avec un rendement de 90 %. Il est caractérisé par ses spectres IR et RMN. De plus, traité par de l'anhydride acétique (1 ml pour 180 mg de 9a), il donne le composé 10a.

2° A partir de 10a, ou 10b - Méthode a: Le dérivé 10a (350 mg) est dissous dans le méthanol (5 ml). Du méthanolate de sodium (0,2 ml, 2M) est ajouté. Après 1 heure à température ambiante, la réaction est arrêtée par addition de résine dowex-50-H+. Après filtration, le produit 13 est obtenu, contaminé par un peu de produit résultant de l'α,β-élimination. La réaction se fait de la même façon à partir de 10 b.

Méthode b: Le dérivé 13 peut être obtenu à partir de 10a ou 10b de la façon décrite pour 12 à partir de 9a ou 9b.

**Exemple 6 A** (voir figure 3)

Application du composé 13 à la synthèse du disaccharide 15

A une solution de monosaccharide 13 (0,215 g; 0,5 mmole) dans le dichlorométhane (3 ml), on ajoute le monosaccharide 14 (0,49 g; 1 mmole) dans du dichlorométhane (3 ml) puis des tamis 4 Å en poudre. On refroidit le mélange à 0°C, puis on ajoute de la sym-collidine (0,16 ml), et du triflate d'argent (0,3 g). Après 1 heure, le mélange est dilué avec du dichlorométhane (50 ml). Les solides sont essorés, puis la solution est lavée avec une solution à 5 % de bicarbonate de sodium, avec de l'eau puis du sulfate acide de potassium à 10 % et à nouveau avec de l'eau. On obtient ainsi, après évaporation 591 mg de résidu. Après purification sur silice dans un mélange toluène/acétone 30/1 (v/v), on récupère 211 mg de disaccharide 15 pur.

**Exemple 7** (voir figure 4)

Préparation du (bromo 2,3-di-O-benzyl-4-O-acétyl-α-D-glucopyranoside) uronate de méthyle (composé 25)

Synthèse du composé 19

A une solution de 16 (32 g; 85,5 mmoles) dans la pyridine (250 ml), on ajoute du chlorure de trityle (28,6 g; 1,2 eq) puis on chauffe à 80°C. Une nouvelle addition de chlorure de trityle (4,6 g; 0,2 eq) est faite après 3 heures de réaction. Lorsque la formation de 17 est complète (c.c.m. silice; méthanol/chloroforme, 1/20, v/v) on refroidit la solution jusqu'à 0°C, puis on ajoute du chlorure de benzoyle (15 ml; 1,5 eq).

Après une nuit, 18 est formé quantitativement. Du méthanol (150 ml) est alors ajouté goutte à goutte au mélange réactionnel qui est ensuite concentré à sec. Le résidu obtenu est repris dans du méthanol (500 ml) contenant de l'acide paratoluène-sulfonique (95 g).

Après 2 heures de réaction, le mélange réactionnel est transvasé dans une ampoule à décanter contenant de l'eau glacée (2 l). Le produit 19 est extrait au chloroforme puis engage tel que dans l'étape suivante.

Une partie de ceproduit a été purifiée. L'analyse du spectre IR confirme la structure. C'est une gomme incolore. $[\alpha]^{20}_D$ - 61° (chloroforme).

Synthèse du composé 23

Le sirop obtenu à l'étape précédente (95 g) est dissous dans l'acétone (1 l), puis à la solution, refroidie à 0°C, on ajoute goutte à goutte, une solution d'oxyde de chrome (52 g) dans l'acide sulfurique 3,5 M (220 ml). Après 2 heures de réaction, le mélange réactionnel est versé dans de l'eau glacée (1 l). Le produit 20 est extrait du chloroforme (5 x 200 ml). La phase chloroformique est lavée jusqu'à pH neutre, séchée et concentrée à sec.

Au résidu obtenu ci-dessus, dissous dans le méthanol (650 ml), on ajoute goutte à goutte de la soude en solution dans l'eau (20 g dans 50 ml), puis on chauffe le mélange à 50°C. Après une nuit, la solution obtenue est concentrée partiellement, puis versée dans l'eau (1,5 l). La phase aqueuse est en suite lavée avec de l'éther puis, après acidification par l'acide chlorhydrique, le produit 21 est extrait à l'éther. La phase éthérée est séchée sur sulfate de sodium, puis concentrée à sec, livrant une masse jaune (50 g) qui contient 20.

Ce réside (50 g) est dissous dans un mélange d'acide acétique et d'acide trifluoroacétique (15/1, v/v, 615 ml). A cette solution, agitée à 100°C, on ajoute de l'eau (160 ml). Après une nuit on évapore à sec et élimine les traces d'acide acétique, évaporation de toluène. Le résidu formé en partie de 21 non hydrolysé et de 22 est dissous dans l'éther (400 ml).

A cette solution, on ajoute à 0°C, une solution éthérée de diazométhane jusqu'à complète méthylation (c.c.m. silice étherhexane, 2/1, v/v). L'excès de diazométhane est alors détruit par l'acide acétique puis le mélange réactionnel est concentré à sec.

Le résidu est purifié sur une colonne de gel de silice (200 g) éluée d'abord par du chloroforme pur, puis par un mélange chloroforme/éther, 3/1, v/v. On obtient ainsi 23 (8,6 g; 22,2 mmoles, 26 % par rapport à 16).

Le dérivé 23 est cristallin p.f. 122 - 123 °C. L'analyse élémentaire et le spectre de RMN confirment sa structure.

Synthèse du composé 24

A une solution de 23 (3,9 g; 10 mmoles) dans la pyridine (50 ml), on ajoute de l'anhydride acétique (4 ml, 42 mmoles). Après 2 heures, le mélange réactionnel est évaporé à sec. On obtient ainsi 24 (4,62 g; 98 %).

Synthèse du composé 25

A une solution de 24 (1,4 g) dans le dichlorométhane 30 ml et l'acétate d'éthyle (3 ml), on ajoute du tétrabromure de titane (1,5 g). La solution est agitée toute la nuit à température ambiante. Après dilution par du dichlorométhane, le mélange réactionnel est versé dans l'eau glacée. La phase organique est lavée avec du bicarbonate à 5 % dans l'eau, séchée et concentrée. Le résidu est chromatographié sur silice (50 g, éther/hexane, 1/1, v/v).

On obtient ainsi le composé 25 (920 mg, 62 %); c'est un sirop incolore $[\alpha]^{20}_D = +97,5°$ (c = 1, chloroforme). L'analyse élémentaire et le spectre de RMN confirment la structure.

**Exemple 7a** (voir figure 5)

Application du composé 25 à la synthèse du disaccharide 27

On effectue cette synthèse à partir des dérivés 25 et 26. Une solution du dérivé 26 (432 mg, 3 mmoles) dans le dichlorométhané (10 ml) est agitée à 0°C en présence de tamis moléculaire 4 Å (0,5 g), de driérite (1 g) et de carbonate d'argent fraîchement préparé (0,42 g). Après refroidissement à 0°C, on ajoute, goutte à goutte, une solution du composé 25 (490 mg, 1 mmole) dans le dichlorométhane (6 ml). La réaction dure deux heures, le mélangé reactionnel est ensuite filtré. Après évaporation à sec et chromatographie sur gel de silice du résidu, (solvant: acétate d'éthyle/chloroforme, 1,6 v/v), on obtient le dérivé 27.

La structure du dérivé 27 est confirmée par son analyse élémentaire et son spectre de RMN. Pouvoir rotatoire: $[\alpha]^{20}_D = 39°$; chloroforme; PF - 156 - 159°C.

**Exemple 8** (voir figure 6)

Préparation du (trichloro acétimidyl 2,3-di-O-benzyl-4-O-acétyl) uronate de méthyle (composé 29)

Synthèse du composé 10a

Le dérivé 10a (11,4 g) est dissous dans un mélange acétone/eau (5/1; v/v; 350 ml). On ajoute à cette solution de l'oxyde mercurique (13,5 g), puis, goutte à goutte, une solution de chlorure mercurique dans le mélange acétone/eau (17 g dans 116 ml). Après 5 minutes, le mélange est filtré puis les solvants sont évaporés. Le résidu, repris par du chloroforme (300 ml) est lavé avec une solution saturée d'iodure de potassium puis avec de l'eau. Après séchage et évaporation à sec, le résidu est purifié sur colonne de gel de silice (200 g

hexane/acétate d'éthyle, 1/1, v/v). On obtient ainsi 28 (6 g; 57,5 %). C'est un solide, p.f. 104 - 106°C. $[\alpha]^{20}_D$: -4,5 (1,2 chloroforme). Le spectre de RMN et l'analyse élémentaire confirment la structure.

Synthèse du composé 22

A une solution du composé 10a (3,46 g; 8 mmoles), et de trichloroacétonitrile (8 ml; 10 eq) dans le dichlrométhane (80 ml), on ajoute de l'hydrure de sodium (135 mg). L'évolution de la réaction est suivie en c.c.m. (chloroforme/acétate d'éthyle; 1/1; v/v). Après filtration, la solution est concentrée à sec, puis le résidu est séché par évaporation de benzène avant d'être engagé dans la réaction avec 29.

**Exemple 8A** (voir également figure 6)

Application du composé 29 à la synthèse du disaccharide 31

Synthèse du disaccharide 31

Le produit 29 obtenu ci-dessus (à partir de 8 mmoles de 28) est dissous dans du dichlorométhane (40 ml) contenant le dérivé 30 (1,1 g; 7,6 mmoles). A cette solution, on ajoute goutte à goutte, une solution de trifluoroéthérate de bore dans le dichlorométhane (0,6 eq). L'évolution de la réaction est contrôlée en c.c.m. (chloroforme/acétate d'éthyle, 6/1, v/v). Du bicarbonate de sodium solide est ajouté jusqu'à neutralisation, puis la phase dichlorométhane est lavée avec de l'eau jusqu'à pH neutre. Après séchage et évaporation des solvants, le sirop obtenu est chromatographié sur gel de silice (200 g, chloroforme/acétate d'éthyle, 8/1, v/v). On obtient ainsi le composé 31 (1,56 g).

L'autre anomère (α-D-glucuronosyl) est également obtenu en quantité moindre (250 mg). 29 est en tout point analogue au produit 31 décrit dans la demande de brevet FR-8 200 621 au nom de la Demanderesse.

**Exemple 9** (voir figure 7)

Préparation du (trichloroacétimidyl - 2,3-di-O-benzyl-4-O-allyl-α-D-glucopyranoside) uronate de méthyle (composé 38)

Synthèse du derive 37

Le composé 32 est allylé de façon classique classique (hydrure de sodium bromure d'allyl dans le diméthylformamide) pour donner le composé 33. Celui-ci est alors soumis à une suite de réactions identiques à celles décrites ci-dessus pour le composé 18, (détritylation, oxydation, hydrolyse, méthylation). A ce stade, le produit est purifié sur une colonne de gel de silice (200 g; chloroforme/acétate d'éthyle; 10/1; v/v). On obtient le composé 37 sous forme de sirop $[\alpha]^{20}_D$ + 23,5° (1, chloroforme). Le spectre IR et l'analyse élémentaire confirment la structure.

Synthèse de 38

Le composé 37, traité (comme il est décrit pour 17), par le trichloroacétonitrile en présence d'hydrure de sodium conduit à l'imidate 38 qui est engagé dans la synthése de 40.

**Exemple 9A** (voir également figure 7)

Application du composé 38 à la préparation du disaccharide 40

Synthèse du disaccharide 40

A une solution de 38 (615 mg; 1,1 mmole) et de 39 (300 mg; 1 mmole) dans le dichlorométhane (5 ml), on ajoute, à 20°C, une solution de trifluoroéthérate de bore (1 mmole) dans le dichlorométhane (10 ml). La réaction est suivie en t.l.c. (acétone/toluène; 1/10: v/v). Le mélange réactionnel est dilué avec du dichlorométhane puis neutralisé avec du bicarbonate de sodium (500 mg). La solution obtenue est lavée avec une solution siturée de chlorure de sodium, séchée, puis concentrée à sec. Le résidu est chromatographié sur colonne de gel de silice (50 g; toluène/éther isopropylique; 4/1; v/v). On obtient ainsi 40 (147 mg; $[\alpha]^{20}_D$ + 11° - chloroforme-) et l'isomère (162 mg; (162 mg; $[\alpha]^{20}_D$ + 48° - 1, chloroforme-). L'analyse élémentaire et l'étude des spectres de RMN confirment les structures des produits.

Les formules des exemples 10 à 12A sont représentées sur la figure 8.

**Exemple 10**

Préparation du prop-1'-ényl 2,3-di-O-benzyl-4-O-chloroacétyl-α-D-glucopyranoside) uronate de méthyle (composé 41)

Cette synthèse est effectuée à partir du composé 13 de l'exemple 6 ou (prop-1'-éthyl-2,3-di-O-benzyl-α-D-glucopyranoside) uronate de méthyle en procédant comme suit:

On dissout 2,8 g du composé 13 dans 30 ml de pyridine (6,56 mmoles). Après refroidissement à 0°C, on ajoute goutte à goutte 10 ml d'une solution de 2 ml de chlorure de chloroacétyle dans 20 ml de dichlorométhane. Après 30 minutes, on évapore à sec, on reprend le résidu par 200 ml de chloroforme, on lave avec une solution à 10 % de KH $SO_4$, puis avec de l'eau, on sèche et on concentre. Le sirop obtenu est chromatographié sur gel de silice (200 g; éluant AcOEt/hexane; 1/3; v/v). On obtient ainsi 2,7 g de composé 41 pur sous forme de sirop (rendement 80 %); $[\alpha^{20}_D = +2°$ (c = 1,5; chloroforme). L'analyse élémentaire et le spectre de RMN confirment la structure attendue.

**Exemple 11**

Préparation du (2 3-di-O-benzyl-4-O-chloroacetyl-α-D-glucopyranose) uronate de méthyle (composé 42)

On dissout 2,7 g (5,3 mmoles) du dérivé 41 dans 80 ml d'un mélange acétone/eau (5/1; v/v). De l'oxyde mercurique (3,1 g) est ajouté suivi d'une solution de chlorure mercurique (3,9 g) dans l'acétone (27 ml). Après 5 minutes, les sels sont éliminés par filtration. Après concentration à sec, le résidu est repris par du chloroforme. La phase chloroformique est lavée avec une solution de KI à 10 % puis avec de l'eau. Après évaporation, le produit est cristallisé dans un mélange acétate d'éthyle/hexane. On obtient 2 g d'un solide de pf 105 - 107°C; $[\alpha]^{20}_D = -4{,}7°$ (eg, 1; chloroforme). L'analyse élémentaire et l'étude en RMN confirment la structure (rendement 80 %).

**Exemple 12**

Préparation du (1-bromo-2,3-di-O-benzyl-4-O-chloroacétyl-α-D-glucopyranosyl) uronate de méthyle (composé 43)

On dissout 2 g (4,30 mmoles) du composé 42 dans 50 ml de dichlorométhane. On ajoute 4,8 ml (34,4 mmoles) de sym-collidine à 0°C suivie de bromure de bromométhylène diméthyl ammonium (17 mmoles) préparé selon HEPBURN D.R. et HUDSON H.R.J. Chem. Soc. Perkin I (1976) 754 - 757. Après 4 heures de réaction, le mélange est dilué par 100 ml de dichlorométhane, puis versé dans de l'eau glacée. Après lavage avec de l'eau glacée, le solvant est évaporé. Après chromatographie sur gel de silice (20 g; éluant hexane/acétate d'éthyle, 2/1; v/v), on obtient 2,06 g de composé 43 sous forme d'un sirop (rendement 90 %). $[\alpha]^{20}_D = +82{,}5°$ (c = 1,5; chloroforme). L'analyse élémentaire et l'étude en RMN confirment la structure.

**Exemple 12A**

Application du composé 43 à la synthèse du disaccharide 45 ou (3,0-acétyl-1,6-anhydro-2-azido-4-O-[(2,3-di-O-benzyl-4-O-chloroacétyl-β-D-glucopyranosyl) uronate de méthyl]-β -D-glucopyranose

A une solution de 870 mg (3,8 mmoles) de composé 44 dans le dichlorométhane, on ajoute 1 g de drièrite (0,5 g de tamis moléculaires 4 Å, en poudre et 0,525 g de carbonate d'argent fraîchement préparé. Après 2 heures d'agitation on ajoute goutte à goutte, à 0°C, 670 mg (1,3 mmoles) du composé 43. Après 6 jours, les solides sont éliminés par filtration. Le sirop obtenu après concentration est chromatographié sur gel de silice (50 g; éluant: chloroforme/acétate d'éthyle; 4/1, v/v). On obtient le disaccharide 1 sous forme de mousse (421 mg; 50 %). $[\alpha]^{20}_D = -17°$ (c = 1; chloroforme). L'analyse élémentaire confirme la structure. L'étude en RMN confirme la configuration β de la liaison interglycosidique.

**Exemple 13** (voir figure 9)

Préparation du (3-O-benzyl-α-L-glucopyranoside) iduronate de méthyle (composé 56)

de formule

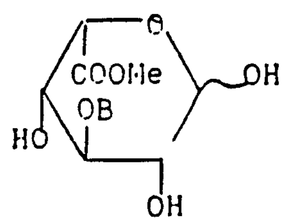

On effectue cette synthèse à partir du composé 46 selon les étapes suivantes:

1) introduction d'un groupe benzoyle en position 5,
2) méthylation de la fonction carboxyle en position 6,
3) isomérisation du groupe OH en position 5,
4) formation du cycle pyranique.

1°) réaction de benzoylation

63 g de 3-O-benzyl-1,2-O-Isopropylidène-α-D-glucofuranoside (composé 46) sont dissous dans 500 ml de pyridine anhydre. On ajoute 85 g de chlorure de trityle et on chauffe à 80° C pendant une heure. On obtient ainsi le composé 47.

Pouvoir rotatoire: $[\alpha]^{20}_D = -34,7°$, chloroforme.

La structure de ce composé a été confirmée par ses spectres IR et RMN, son analyse élémentaire est correcte.

Le mélange est en suite refroidi à 0° C et on ajoute 45 ml de chlorure de benzoyle. Après une nuit, on détruit l'excès des réactifs par addition de 300 ml de méthanol. Le mélange obtenu, évaporé à sec, est repris par du chloroforme. La phase chloroformique est lavée à l'eau, séchée sur sulfate de sodium et concentrée. On obtient ainsi le composé 48.

Le sirop obtenu est dissous dans 400 ml de chloroforme. Après addition de 100 ml d'une solution d'acide paratoluène-sulfonique 5 M dans le méthanol, la solution est laissée à 4° C pendant une nuit. On obtient, après lavage à l'eau de la phase organique, 215 g d'un mélange. Le composé est obtenu par chromatographie de ce mélange sur gel de silice dans le solvant éther-hexane 2/1 (v/v). On obtient ainsi 36 g du composé 49.

Pouvoir rotatoire: $[\alpha]^{20}_D = 65,3°$, chloroforme.

La structure du composé 49 a été confirmée par ses spectres IR et RMN.

2°) méthylation de la fonction carboxyle en position 6

Le composé 49 (1,88 g) est dissous dans l'acétone (20 ml). On ajoute, goutte à goutte à -50° C 3,5 ml d'une solution de $CrO_3$ (13 g) dans $H_2SO_4$, 3,5 M (29 ml). On laisse la température remonter et on laisse une heure dans ces conditions. Le mélange réactionnel est alors versé dans la glace et le produit est extrait au chloroforme. Après lavage à l'eau et séchage, on évapore à sec. On obtient le composé 50.

Le mélange obtenu est dissous dans du méthanol (20 ml), on ajoute ensuite 10 ml de soude 1 N et on laisse une nuit à température ambiante. Le mélange réactionnel est alors passé au travers d'une colonne (25 ml) de résine Dowex 50 sous forme H+ au préalable rincée avec du méthanol. Le produit est obtenu par concentration de l'éluat. On obtient ainsi le composé 51.

Ce composé est dissous dans l'éther et méthylé de façon classique par le diazométhane. On obtient, après évaporation, le composé 52 (1,08 g; 70,4 %).

Pouvoir rotatoire $[\alpha]^{20}_D = -27°$, chloroforme.

L'analyse élémentaire trouvée du composé 52 est correcte. Sa structure est de plus confirmée par ses spectres IR et RMN.

3°) isomérisation du groupe -OH en position 5

A une solution d'anhydride triflique (0,8 ml) dans le dichlorométhane (16 ml), refroidie à -20° C, on ajoute goutte à goutte une solution de pyridine (0,8 ml) dans le dichlorométhane (8 ml). On ajoute ensuite, à -10° C, goutte à goutte, 800 mg de composé 52 dissous dans du dichlorométhane (8 ml). Après une heure à -50° C, le mélange réactionnel est versé dans un mélange d'eau et de glace (8 ml) contenant 160 mg de bicarbonate de sodium. On agite jusqu'à séparation des deux phases organique et aqueuse. La phase organique est lavée par HCl 3 %, $H_2O$, NaCl saturé, séchée et concentrée. On obtient ainsi le composé 53.

Le sirop est repris par du DMF (10 ml). On ajoute du trifluoroacétate de sodium (1,6 g) et chauffe à 80°C pendant 3 heures. On obtient ainsi le composé 54.

Après évaporation, reprise par du dichlorométhane, lavage à l'eau et séchage, le résidu est repris par du méthanol puis le solvant est évaporé après une heure. Après chromatographie sur colonne dans le solvant éther-hexane 2/1, on obtient le composé 55 (450 mg: 56,2 %).

Pouvoir rotatoire: $[\alpha]^{20}_D$ = -33° chloroforme.

La structure du composé 55 est confirmée par ses spéctres IR et RMN. L'analyse élémentaire trouvée est correcte.

4°) formation du cycle pyranique

Cette synthèse est effectuée à partir du composé 55. Le composé 55 (200 mg) est dissous dans un mélange acide trifluoroacétique/eau 9/1.). Après 15 minutes, les solvants sont évaporés. Le résidu est cristallisé dans de l'acétate d'éthyle/hexane. On obtient ainsi 110 mg de compose 56.

Les caractéristiques de ce dérivé sont les suivantes:

- spectre IR: dans $CHCl_3$, $\gamma$ en $cm^{-1}$: 3450(OH), 3080, 3060, 3030 ($CH_2$: benzyle) et 1740 ($COOCH_3$)
- spectre RMN: $\delta$ en ppm par rapport au TMS: 3,75 (s, 3H+, COOMe 4,98 (1H+), 7,30 (s, 5H+, $C_6H_5$)
- pouvoir rotatoire: $[\alpha]^{20}_D$ = +13°, méthanol,

| - analyse élémentaire pour $C_{14}H_{18}O_7$ | calculé | trouvé |
|---|---|---|
| C........................ | 56,37 | 56,17 |
| H........................ | 6,08 | 5,85 |
| - P.F.................... | 125 - 126°C | |

**Exemple 14** (voir figure 10)

Préparation des composés 57 et 58 ou 1,2,4-tri-O-acétyl-3-O-benzyl-α,β-L-méthyl idopyranuronate

Une solution du composé 56 (3 g) préparé selon la demande de brevet FR No 8 200 621 au nom de la Demanderesse dans un mélange de pyridine anhydre (20 ml) et d'anhydride acétique (10 ml) est agitée à 0°C, à l'abri de l'humidité, pendant 5 h. Le mélange réactionnel est évaporé à sec, évaporé avec du toluène (4 x 20 ml), et séché sous vide. Le résidu est chromatographié sur une colonne de gel de silice (150 g). L'élution par le mélange toluène : acétate d'éthyle (4 : 1, v/v) donne, par ordre d'élution:

- une fraction de tête composée de dérivés furanniques,
- le composé 58 (anomère α), sirop, (170 mg, 4 %), $[\alpha]^{20}_D$ = -43°; (c: 1, chloroforme), R.M.N. ($CDCl_3$) : $\delta$ : 6,23 (s, 1H, H-1).
- le composé 57 (anomère β), cristallisant dans un mélange éther-hexane, (2,688 g, 63 %), P.F.: 112 - 113°C, $[\alpha]^{20}_D$ = +9° (c : 1, chloroforme) R.M.N. ($CDCl_3$) : $\delta$ : 6,08 (d, 1H, H-1, $J_{1,2}$ : 1,5 Hz).

Les anomères α et β 57 et 58 ne sont pas séparés lorsqu'on procède à la succession des synthèses décrites. Leur mélange est utilisé directement sous forme de sirop pour les réactions ultérieures.

**Exemple 15** (voir également figure 10)

Préparation du bromure de 2,4-di-O-acétyl-3-O-benzyl-β-L-méthyl idopyranuronyle (composé 59)

Un mélange d'acétates 57 et 58 (212 mg, 0,5 ml) est dissous dans du dichlorométhane anhydre (5 ml) et de l'acétate d'éthyle anhydre (0,5 ml). Du tétrabromure de titane (250 mg, 0,7 mM) est ajouté en une seule fois, et le mélange réactionnel est agité 24 b à la température ambiante à l'abri de l'humidité. Après refroidissement à 0°C et dilution avec du dichlorométhane, la phase organique est lavée avec de l'eau glacée (3 fois), séchée (sulfate de sodium), filtrée et évaporée pour donner 59 sous forme d'un sirop légèrement coloré (217 mg, 96 %). R.M.N. ($CDCl_3$) : $\delta$ : 6,41 (s, 1H, H-1). Ce composé, très instable est immédiatement engagé dans les réactions suivantes.

Les formules des composés des exemples 16 à 19A sont représentées sur les figures 10 et 11.

**Exemple 16**

Préparation du 4-O-acétyl-3-O-benzyl-1,2-O-méthoxyéthylidène-α-L-méthyl idopyranuronate (composé 60)

Une solution du bromure 59 (fraîchement préparé à partir de 0,425 g, 1 mM de mélange d'acétates 57 et 58) dans du dichlorométhane anhydre (10 ml) est agitée à la température ambiante sous atmosphère d'argon sec. De la sym-collidine (0,66 ml, 5 mM) et du méthanol anhydre (0,40 ml, 10 mM) sont successivement ajoutés, et le mélange réactionnel est agité 20 h dans ces conditions. Après dilution avec du dichlorométhane (50 ml), la phase organique est lavée avec une solution saturée aqueuse d'hydrogénocarbonate de sodium, avec de l'eau, séchée (sulfate de sodium), filtrée et évaporée. Le résidu est chromatographié sur une colonne de gel de silice (20 g). L'élution par le mélange hexane : acétate d'éthyle (3 : 2 v/v, contenant 0,5 % de triéthylamine) donne 60 sous forme d'un sirop pur (302 mg, 76 % à partir de 57 et 58), $[\alpha]^{20}_D$ = -21° (c: 1, chloroforme), R.M.N. ($CDCl_3$) : δ : 5,52 (d, 1H, H-1, $J_{1,2}$ : 3 Hz).

**Exemple 17**

Préparation du 4-O-acétyl-3-O-benzyl-1,2-O-tert.-butoxyéthylidène-α-L-méthyl idopyranuronate (composé 61)

Une solution de bromure 59 (fraîchement préparé à partir de 2,122 g, 5 mM, de mélange d'acétates 57 et 58 dans du dichlorométhane anhydre (20 ml) est agitée à la température ambiante sous atmosphère d'argon sec. De la sym-collidine (2,65 ml, 20 mM) et du tert.-butanol anhydre (3 ml, 30 mM) sont successivement ajoutés et le mélange réactionnel est agité 15 h dans ces conditions. Après traitement identique à celui décrit pour le composé 60, le résidu est chromatographié sur une colonne de gel de silice (120 g). L'élution par le mélange hexane : acétate d'éthyle (2 : 1, v/v contenant 0,5 % de triéthylamine) donne 61 sous forme d'un sirop pur (1,542 g, 70 % à partir de 57 et 58, $[\alpha]^{20}_D$ = -23° (c: 1, chloroforme), R.M.N. ($CDCl_3$) : δ : 5,48 (d, 1H, H-1, $J_{1-2}$ : 2,5 Hz).

**Exemple 18**

Préparation du 3-O-benzyl-1,2-tert-butoxyéthylidène-α-L-méthyl idopyranuronate - (composé 62)

Une solution de l'orthoester 61 (484 mg, 1,1 mM) dans le méthanol anhydre (15 ml) est refroidie à -20°C sous agitation et atmosphère d'argon sec. Du carbonate de potassium anhydre (60 mg) est ajouté, le mélange réactionnel est agité 5 h dans ces conditions. Les solides sont essorés, le filtrat est évaporé et le résidu est repris dans du chloroforme (50 ml). La phase organique est lavée avec de l'eau glacée (3 fois) séchée (sulfate de sodium), filtrée et évaporée. Le résidu est chromatographié rapidement sur une colonne de gel de silice (25 g). L'élution par le mélange hexane : acétate d'éthyle (2 : 1, v/v contenant 0,5 % de triéthylamine), donne, par ordre d'élution:
- le composé insaturé 64 (31 mg, 7 %) sirop, $[\alpha]^{20}_D$ = +103° (c: 1, chloroforme), R.M.N. ($CDCl_3$) : δ : 6,27 (d.ded., 1H, H-4, $J_{3,4}$ : 5 Hz, $J_{2,4}$ : 1 Hz), 5,67 (d, 1H, H-1, $J_{1-2}$ : 4 Hz).
- une fraction principale (271 mg, 62 %), qui est cristallisée dans un mélange éther-hexane pour donner 62 (123 mg, 28 %), P.F.: 68 - 69°C; $[\alpha]^{20}_D$ = -19° (c: 1, chloroforme), R.M.N. ($CDCl_3$) : δ : 5,41 (d, 1H, H-1, $J_{1,2}$ : 2 Hz), 2,85 (d, 1H, CH-4, J : 12 Hz, échangé avec $D_2O$).

Au cours de la chromatographie sur silice, et lors des essais de cristallisation de 62, un composé nouveau de Rf légèrement supérieur à celui de 13 apparaît. Une chromatographie sur gel de silice des eaux-mères de cristallisation de 62 permet d'isoler quelques fractions pures de ce nouveau composé 65 (41 mg, 11 %), sirop, $[\alpha]^{20}_D$ = +21° (c: 1, chloroforme), R.M.N.($CDCl_3$) : δ : 5,83 (d, 1H, H-1, $J_{1,2}$ : 4,5 Hz).

Dans le cadre de la succession des synthèses envisagées selon l'invention, afin d'éviter la formation de 65 le sirop brut de 62 n'est pas chromatographié, mais utilisé immédiatement pour la réaction suivante.

**Exemple 19**

3-O-benzyl-4-O-chloroacétyl-1,2-O-tert-butoxyéthylidène-α-L-méthyl idopyranuronate (composé 63)

Une solution de l'orthoester 62 (220 mg, 0,5 mM) dans le méthanol anhydre (10 ml) est refroidie à -20°C sous agitation et atmosphère d'argon sec. Du carbonate de potassium anhydre (40 mg) est ajouté et le mélange réactionnel est agité pendant 5 h dans ces conditions. Les solides sont essorés, le filtrat est évaporé et le résidu est repris dans du chloroforme (50 ml). La phase organique est lavée rapidement avec de l'eau glacée (3 fois), séchée (sulfate de sodium), filtrée et évaporée. Le résidu est immédiatement dissous dans de la pyridine anhydre (4 ml) et du dichlorométhane anhydre (2 ml). Après refroidissement à -20°C sous atmosphère d'argon

sec, une solution de chlorure de chloroacétyle (0,1 ml, 1,25 mM, fraîchement distillé) dans le dichlorométhane anhydre (1 ml) est ajoutée goutte à goutte. Le mélange réactionnel est agité dans ces conditions pendant 30 mn, puis versé dans un mélange eau-glace (100 ml). Après agitation pendant 15 mn, le mélange est extrait avec du chloroforme (3 x 20 ml). Les phases organiques sont lavées avec de l'eau glacée, avec une solution aqueuse à 2 % d'hydrogénocarbonate de sodium, avec de l'eau, séchées (sulfate de sodium), filtrées et évaporées. Le résidu est chromatographié rapidement sur une colonne de gel de silice (12 g). L'élution par le melange hexane: acétate d'éthyle (5 : 2, v/v, contenant 0,2 % de triéthylamine) donne, par ordre d'élution:
- le composé insaturé 64 (15 mg, 8 %),
l'orthoester 63 sirop (145 mg, 61 % à partir de 12), $[\alpha]^{20}_D = +19°$ (c: 1, chloroforme), R.M.N. ($CDCl_3$) : δ : 5,45 (d, 1H, H-1, $J_{1,2}$ : 2,5 Hz), 5,24 (d.de d., 1H, H-4, $J_{3,4}$ : 2,5 Hz, $J_{4,5}$ : 1,5 Hz), 4,00 (s, 2H, Cl-$CH_2$-COO-).

**Exemple 19A** (voir figure 12)

Application du composé 63 à la synthèse du disaccharide 67

Composé: Méthyl 6-O-benzoyl-3-O-benzyl-2-benzyloxycarbonylamino-2-désoxy-4-O-(2,4-di-O-acétyl-3-O-benzyl-α-L-méthyl idopyranuronyl)-α-D-glucopyranoside.

Une solution de l'orthoester 63 (80 mg, 0,2 mM) et de l'alcool 66 (52 mg, 0,1 mM) dans du chlorobenzène anhydre (8 ml) est chauffée à 140°C sous agitation et léger courant d'argon sec. Après distillation lente de 6 ml de solvant, une solution de perchlorate de 2,6 diméthylpyridinium (0,002 mM fraîchement préparé selon N.K. KOCHETKOV, A.F. BOCHKOV, T.A. SOKOLOVSKAIA et V.J. SNIATKOVA, Carbonhdr. Res., 16 (1971) 17 - 27, dans le chlorobenzène (2 ml) est ajoutée goutte à goutte en 15 mn. avec distillation simultanée de solvant (2 ml). Le mélange réactionnel est alors agité pendant 1 h, dans ces conditions, avec addition de solvant frais (10 ml) et distillation simultanée de telle sorte que le volume réactionnel reste constant et égal à 2 ml. Après refroidissement et dilution avec du chloroforme, la phase organique est lavée avec une solution saturée d'hydrogénocarbonate de sodium, avec de l'eau, séchée (sulfate de sodium), filtrée et évaporée. Le résidu est chromatographié sur une colonne de gel de silice (15 g). L'élution par le mélange hexane : acétate d'éthyle (4 : 3, v/v) donne, par ordre d'élution:
- le produit de départ 66 (20 mg, 38 %),
- une fraction homogène en chromatographie sur couche mince (54 mg). Le spectre de R.M.N. de cette fraction montre la présence de plusieurs signaux O-méthyl (δ : 3,35 - 3,50) dus aux méthyl glycosides provenant du réarrangement de l'orthoester 63. Cette fraction est cristallisée dans un mélange éthanol-eau, et recristallisée dans un mélange acétate d'éthyle-hexane pour donner 67 (44 mg, 50 %), P.F.: 120 - 121°C, $[\alpha]^{20}_D = +17°$ (c : 1, chloroforme), R.M.N. ($CDCl_3$): conforme à la structure attendue.

**Exemple 20** (voir figure 13)

Préparation du (méthyl-2,3-di-O-benzyl-α-L-glucolpyranoside) iduronate de méthyle (composé 79)

Cette synthèse est effectuée selon les étapes 1 à 7 suivantes.

**Etape 1:** synthèse du monosaccharide 69

On prépare ce monosaccharide à partir du composé 68 obtenu selon la technique de N.L. Holder et B. Fraser-Reid, Canadian Journal of Chemistry, 51 (1973) page 3357. A une solution du composé 68 (1 g, 12,67 mM) dans le dichlorométhane (20 ml), on ajoute du chlorure de tosyle (0,55 g), puis de la diméthylaminopyridine (16 mg) et enfin, de la triéthylamine (0,7 ml). Après agitation sous courant d'azote à l'abri de l'humidité, pendant environ 14 heures, la réaction est arrêtée par addition de glace et d'eau. Après dilution du mélange réactionnel avec du dichlorométhane (50 ml), la phase dichlorométhane est lavée avec de l'acide chlorhydrique 2 M, puis une solution saturée de bicarbonate de sodium, et enfin avec de l'eau jusqu'à pH neutre. Après séchage et évaporation on obtient un résidu, à savoir le dérivé 69 (1,4 g, 97 %) qui est engagé tel quel dans la synthèse du dérivé 70.

**Etape 2:** synthèse du dérivé 70

Le monosaccharide 69 (31,8 g) et de l'iodure de sodium (39 g) sont dissous dans de l'acétonitrile (250 ml), puis la solution est portée à reflux pendant 3 heures. Après refroidissement du mélange réactionnel, le précipité blanc formé est filtré. Le filtrat est concentré, le résidu est repris par du chloroforme, puis la phase chloroformique est lavée avec de l'eau jusqu'à pH neutre, séchée sur sulfate de sodium et concentrée à sec. On obtient un sirop qui est chromatographié sur une colonne de gel de silice (200 g, éther-hexane, 1/1, v/v). On

obtient ainsi le dérivé iodé 70 (24,7 g, 71,5 %). $[\alpha]^{D}_{20}$ = 24° (1, chloroforme).

Le spectre infrarouge, le spectre de RMN et l'analyse elementaire confirment la structure de 70.

**Etape 3:** synthèse du dérivé 71

A une solution du dérivé 70 dans de la pyridine anhydre (200 ml), on ajoute de l'anhydride acétique (43 ml). Après environ 14 heures sous agitation, la réaction est terminée. Le mélange réactionnel est concentré à sec, puis le résidu est purifié sur une colonne de gel de silice, sous pression, dans un solvant acétate d'éthyle-hexane (1/6, v/v). Les fractions pures sont regroupées. On obtient ainsi le produit 71 (16,4 g, 70 %). Ce produit se présente sous forme d'un sirop. $[\alpha]^{D}_{20}$ = +4,5° (1,3, chloroforme). L'analyse élémentaire ainsi que l'analyse du spectre infrarouge confirment la structure.

**Etape 4:** synthèse de 72

A une solution du dérivé 71 (4 g) dans de la pyridine (100 ml), refroidie à 0°C, on ajoute du fluorure d'argent (AgF, 6,9 g). Après deux heures et demie, le mélange réactionnel est versé dans un mélange contenant du chloroforme et de l'éther (1/4, v/v, 1 l). La suspension obtenue est passée au travers d'un filtre plissé. Le filtrat est concentré à sec, puis le résidu est repris dans du chloroforme (500 ml). La phase chloroformique est lavée avec du sulfate acide de potassium en solution à 10 % dans l'eau, puis avec de l'eau jusqu'à pH neutre. Après séchage sur sulfate de sodium et concentration à sec, on obtient un résidu (2,7 g), qui est chromatographié sur une colonne de silice (200 g) (éluant : acétate d'éthyle-hexane, 1/4, v/v). Les fractions contenant le produit 72 sont regroupées et après évaporation des solvants, on obtient un produit cristallin (1,62 g, 54 %).

P.F.: 81 - 82°C, $[\alpha]^{D}_{25}$ = -20° (1, chloroforme). L'analyse du spectre infrarouge, l'analyse élémentaire et l'analyse du spectre de résonnance magnétique nucléaire confirment la structure du compose 72.

**Etape 5:** synthèse du dérivé 73

Le produit 72 (2 g) est dissous dans du méthanol (20 ml) et du chloroforme (20 ml). A cette solution, on ajoute du méthanolate de sodium (2 M, 2 ml). Après 1,5 heure, la réaction de désacétylation est terminée. Le mélange réactionnel est dilué avec du chloroforme. La phase chloroformique est lavée avec de l'eau jusqu'à pH neutre, séchée, puis évaporée à sec. On obtient ainsi un résidu, le composé 72 (1,8 g, 100 %). Il est immédiatement dissous dans du tétrahydrofuranne (50 ml), puis de l'hydrure de bore ($BH_3$, 1 M) dans le tétrahydrofuranne; (10 ml) est ensuite ajouté. Après une heure de réaction, l'excès d'hydrure de bore est détruit par addition d'éthanol. A la fin du dégagement gazeux, le mélange réactionnel est dilué par addition de tétrahydrofuranne (100 ml). De la soude 3 M (12 ml) est ensuite ajoutée, suivie d'eau oxygénée (120 volumes, 8 ml). Après 2 heures de chauffage à 50°C, la réaction est arrêtée. La solution est versée dans du chloroforme (500 ml), puis la phase organique ainsi obtenue est lavée avec de l'eau, de l'acide chlorhydrique 2 M, puis enfin avec de l'eau jusqu'à pH neutre. On obtient ainsi une phase chloroformique très laiteuse, qui devient limpide au cours du séchage sur sulfate de sodium. Après filtration, le chloroforme est évaporé puis le résidu obtenu est chromatographié sur silice (200 g chloroforme-méthanol, 30/1, v/v).

On obtient ainsi le dérivé de l'idose 73 (1,05 g 55 %). Ce produit se présente sous forme d'un sirop. $[\alpha]^{D}_{20}$ = +85,5° (1, chloroforme).

L'analyse élémentaire ainsi que l'analyse en R.M.N. confirment la structure attendue.

**Etape 6:** synthèse du dérivé 76

Cette synthèse est effectuée à partir du dérivé 73 en une seule étape (les intermédiaires 74 et 75 ne sont pas isolés). A une solution du dérivé 73 (2,25 g, 6 mM) dans le dichlorométhane (50 ml), on ajoute successivement de la diméthylaminopyridine (60 mg; 0,24 mM) de la triethylamine (1,7 ml; 12 mM) et du chlorure de trityle (2,5 g; 9 mM). Après environ 14 heures, la réaction est terminée. On obtient ainsi en solution le dérivé 74. On ajoute alors au mélange réactionnel de la diméthylaminopyridine (150 mg), de la triéthylamine (1,7 ml) et du chlorure de benzoyle (1,05 ml). Après 6 jours, le dichlorométhane est éliminé par passage d'un courant d'azote et remplacé par du diméthylformamide (40 ml). Le mélange réactionnel est chauffé à 70°C pendant une nuit. On ajoute alors à nouveau du chlorure de benzoyle (1 ml) et de la triéthylamine (1,7 ml), puis on maintient le chauffage à 70°C pendant 2 jours. Le diméthylformamide est ensuite évaporé, puis le résidu est repris par du chloroforme, la phase chloroformique est lavée avec de l'eau, avec une solution saturée de bicarbonate de sodium, puis avec une solution d'acide chlorhydrique 2 M et enfin avec de l'eau jusqu'à pH neutre. Après séchage, le chloroforme est évaporé, ce qui permet d'obtenir le composé 75.

Celui-ci est immédiatement soumis à une réaction pour éliminer le groupe trityle afin d'obtenir le derive 76. Le résidu contenant le dérivé 75 est dissous dans 25 ml de chloroforme et on ajoute à cette solution 10 ml d'une solution d'acide paratoluènesulfonique monohydrate dans le méthanol (1 M). Après 4 heures de réaction à température ambiante, la réaction est terminée. Le mélange réactionnel est alors dilué avec du chloroforme, lavé avec de l'eau, séché puis évaporé à sec. Le résidu obtenu est chromatographié sur gel de silice (200 g, éther-hexane, 3/1, v/v). Le dérivé 76 est ainsi obtenu à l'état pur (1,5 g; 52 %). Ce dérivé se présente sous forme d'un sirop. $[\alpha]^{D}_{20}$ = -8° (1, chloroforme).

L'analyse du spectre infrarouge et du spectre R.M.N. confirment la structure du produit attendu.

**Etape 7:** synthèse du composé 79

Cette synthèse est effectuée directement à partir du dérivé 76 sans isoler les intermédiaires 77 et 78. A la solution du composé 76 (1,2 g) dans l'acétone (20 ml), on ajoute, goutte à goutte, après refroidissement à 0°C, une solution (2,9 ml) d'oxyde de chrome ($CrO_3$; 1,17 g) dans l'acide sulfurique 3,5 M (5 ml). Après 30 minutes d'agitation à 0°C, la température est ramenée à l'ambiante. La réaction évolue pendant 3 heures. Le mélange réactionnel est ensuite versé dans une ampoule à décanter contenant de l'eau glacée (100 ml). Le produit formé est extrait par du chloroforme (3 x 50 ml). La phase chloroformique est lavée avec de l'eau jusqu'à pH neutre, puis séchée sur sulfate de sodium, filtrée et concentrée à sec. Le résidu obtenu (le composé 77) est dissous dans du méthanol (130 ml). On ajoute à cette solution de la soude 3 M (17 ml) puis on laisse le mélange sous agitation pendant environ 14 heures. Après acidification par l'acide sulfurique, le composé 78 est extrait à l'éther, puis immédiatement méthylé par du diazométhane selon la méthode classique pour donner le composé 79.

Après évaporation de l'éther, le composé 79 est obtenu pur au moyen d'une chromatographie sur gel de silice (50 g: éther-hexane; 4/1; v/v). Les fractions pures contenant le dérivé 79 sont rassemblées et les solvants sont éliminés. On obtient ainsi le dérivé 79 de l'acide iduronique (587 mg, 59 % par rapport au dérivé 76. Ce produit se présente sous forme d'un sirop. $[\alpha]^{D}_{25}$ = +98° (2,65, chloroforme).

L'analyse en R.M.N., l'analyse en infrarouge et l'analyse élémentaire confirment la structure attendue.

**Exemple 20A** (voir figure 13)

Application du composé 79 à la synthèse du disaccharide 81

Cette synthèse s'effectue à partir du monosaccharide 79 préparé comme ci-dessus et du monosaccharide 80 préparé selon la technique de H. Paulsen et W. Stenzel, chemische Berichte 111 (1978) 2234 - 2247.

A une solution du composé 79, (200 mg, 0,5 mM) dans le dichlorométhane (10 ml) on ajoute successivement le composé 80 (0,450 g) de la sym-collidine (150 µl) et du triflate d'argent (260 mg).

Le mélange réactionnel est maintenu à 0° C sous courant d'azote et sous agitation à l'abri de l'humidité et de la lumière pendant 3 heures.

Il est ensuite dilué avec du dichlorométhane (100 ml) puis les solides sont éliminés par filtration sur filtres plissés. La solution obtenue est lavée avec une solution saturée de bicarbonate de sodium avec de l'eau et avec de l'acide sulfurique 2 M, puis à nouveau avec de l'eau jusqu'à pH neutre.

Après séchage sur sulfate de sodium et évaporation du dichlorométhane, le résidu obtenu est chromatographié sur gel de silice (50 g; chloroforme/acétate d'éthyle; 15/1; v/v).

On obtient ainsi le dérivé 81 pur (327 mg, 82 %). Le produit se présente sous forme d'un sirop. $[\alpha]^{D}_{20}$ = +57° (1, chloroforme).

L'analyse en R.M.N. de même que l'analyse élémentaire confirment la structure et l'anomérie du disaccharide 81.

**Exemple 21** Synthèse du trisaccharide 86 de formule

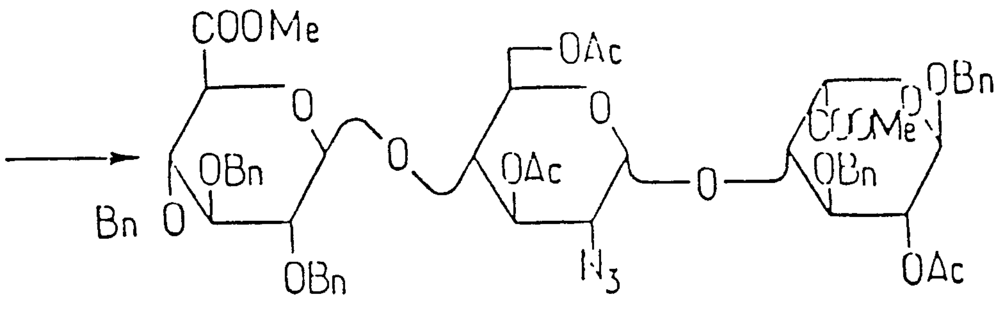

(86)

Cette synthèse est réalisée en 3 étapes (voir figure 15). Tout d'abord, on procède à la glycosylation de l'orthoester d'un dérivé d'acide L-iduronique. On élimine ensuite sélectivement le groupe monochloroacétyle, puis on fait réagir l'un des alcools formés avec un disaccharide.

1) Glycosylation de l'orthoester 63 par l'alcool benzylique

Une solution de l'orthoester 63 (118 mg, 0,25 mM) obtenu comme décrit ci-dessus et d'alcool benzylique (0,15 ml, 15 mM, fraîchement distillé) dans du chlorobenzène anhydre (10 ml) est chauffée à 140°C à l'abri de l'humidité. Après distillation lente de 8 ml de solvant, une solution de perchlorate de 2,6-diméthylpyridinium (2,5 µM) dans le chlorobenzène (2 ml) est ajoutée goutte à goutte en 30 mn avec distillation simultanée de solvant (2 ml). Le mélange réactionnel est alors agité 30 mn dans ces conditions, avec addition goutte à goutte de solvant frais et distillation simultanée, de telle sorte que le volume réactionnel reste constant et égal à environ 2 ml. Après refroidissement et dilution avec du chloroforme (50 ml), la phase organique est lavée avec une solution aqueuse à 5 % d'hydrogénocarbonate de sodium, avec de l'eau, séchée (sulfate de sodium) filtrée et évaporée.

Le résidu est chromatographié sur une colonne de gel de silice (8 g). L'élution par le mélange hexane-acétate d'éthyle (2 : 1, v/v) permet d'obtenir une fraction contenant le mélange 82 de glycosides α et β qui n'ont pas été séparés à ce stade (102 mg, 81 %), R.M.N. (90 MHz, $CDCl_3$) : δ : 7,30 (m, 10 H, 2 Ph, 3,98 (s, 2H, $Cl-CH_2-CO$), 3,74 (s, 3H, COOMe), 2,08 et 2,03 (2s, 3H au total, OAc forme β et α; β : α ≃ 2 : 1).

2) O-démonochloroacétylation sélective

Une solution du mélange 82 précédent (102 mg) dans de la pyridine (5 ml) et de l'éthanol absolu (1 ml) est chauffée à 100°C pendant 20 min. en présence de thiourée (25 mg). Après refroidissement, le mélange réactionnel est évaporé à sec et le résidu est repris par un mélange eau-chloroforme (1 : 1, v/v, 50 ml). La phase organique est lavée avec de l'eau, séchée (sulfate de sodium), filtrée et évaporée.

Le résidu est chromatographié sur une colonne de gel de silice (10 g). L'élution par le mélange acétate d'éthyle-hexane (4 : 3, v/v) permet d'isoler (par ordre d'élution):

- le glycoside β 83 (26 mg, 25 %), sirop incolore, $[\alpha]_D$ + 70° (c1, chloroforme) R.M.N. (90 MHz, $CdCl_3$) : δ : 7,30 (m, 10H, 2 Ph); 5,05 (m, 1H, $H_2$); 4,90 (d, 1H, $H_1$, 1,2 J = 2Hz); 3,78 (s, 3H, COOMe); 3,12 (1H, OH, échangé avec $D_2O$); 2,05 (s, 3H, OAc).
- le glycoside α 84 (54 mg, 50 % à partir de 63) sirop incolore, $[\alpha]^{20}_D$ - 65° (c 1, chloroforme) R.M.N. (90 MHz, $CdCl_3$) : δ : 7,30 (m, 10 H, 2 Ph); 5,05 (2 H, $H_1$ et $H_2$, constantes de couplage très faibles pour $J_{1,2} \leqslant 1$ Hz); 3,78 (s, 3 H, COOMe); 2,80 (1 H, OH, échangé avec $D_2O$); 2,06 (s, 3 H, OAc).

3) Glycosylation de l'alcool 84 à l'aide du disaccharide 85.

Une solution de l'alcool 84 (22 mg, 50 µM), et du bromure 85 (57 mg, 70 µM) dans du dichlorméthane anhydre (1,5 ml) est agitée à l'abri de la lumière et de l'humidité en présence de tamis moléculaire 4 Å (poudre, 50 mg). Le mélange réactionnel est refroidi à -20°C et de la sym-collidine (110 µl) et du triflate d'argent (26 mg, 100 µM) sont ajoutés successivement. Le mélange réactionnel est agité 2 h dans ces conditions, dilué avec du dichlorométhane (50 ml) les solides sont essorés et le filtrat est lavé avec une solution aqueuse glacée d'HCl 0,1 M, avec de l'eau, avec une solution aqueuse à 5 % d'hydrogénocarbonate de sodium, avec de l'eau, séché (sulfate de sodium), filtré et évaporé.

Le résidu est chromatographié sur une colonne de gel de silice (8 g, gel 230 - 400 mesh). L'élution par le mélange toluène-acétate d'éthyle (5 : 1, v/v) permet d'isoler le trisaccharide 86 sous forme d'un sirop incolore (50 mg, 86 %).

Le spectre R.M.N. (270 MHz, $CDCl_3$) est conforme avec la structure attendue.

**Exemple 22** (voir figure 16)

Application du dérivé d'acide L-iduronique 87 de formule

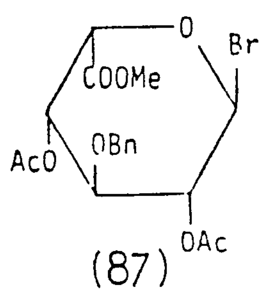

(87)

à la synthèse de glycosides utilisables en tant que substrats d'enzymes, de formule:

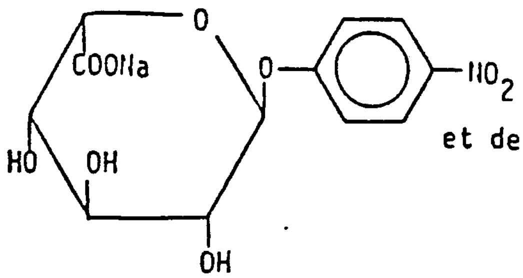

et de

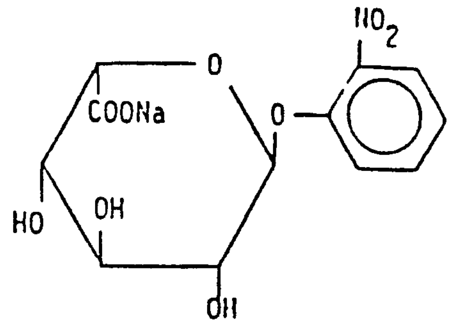

Synthèse du composé 87

Le composé 87 (demande de brevet FR No 8 201 575; 900 mg; 2 mmoles) est dissous dans du dichloroéthane (20 ml). On ajoute alors du tributyl Stannyl Phénol (88; 2,5 μmoles) puis du chlorure stannique (2,5 mmoles). Après 5 heures, le mélange réactionnel est dilué avec du dichlorométhane, lavé avec une solution de bicarbonate desodium, puis avec de l'eau. Le résidu obtenu après séchage et évaporation des solvants (1,03 g) est directement hydrogéné. Pour cela, il est dissous dans du méthanol, puis la solution est agitée sous atmosphère d'hydrogène en présencé de Pd/C (5 %; 0,5 g) pendant 3 heures. Le catalyseur est alors éliminé par filtration puis la solution est concentrée à sec. Le résidu est acétylé par l'anhydride acétique en présence de pyridine, livrant ainsi 89 (420 mg) purifie sur une colonne de gel de silice (100 g; Ether/hexane; 3/1; v/v). Le spectre de R.M.N. et l'analyse élémentaire confirment la structure de 89.

Synthèse des composés 93 et 95

A une solution de 89 (400 mg) dissous dans l'acide acétique glacial (2,5 ml) on ajoute goutte à goutte, sous agitation et à 0°C, un mélange (1 ml) constitué d'anhydride acétique (16 volumes) et d'acide nitrique (5 volumes). Après deux heures à 35°C, le mélange réactionnel est versé dans de l'eau glacée puis les produits formés (90 et 91) sont extraits au chloroforme. La phase chloroformique est lavée avec du bicarbonate à 5 % dans l'eau, séchée puis concentrée à sec. Les produits 90 et 91 formés dans un rapport d'environ 2/1, sont séparés par chromatographie sur gel de silice (25 g; éther/hexane, 1/1, v/v). On obtient ainsi 90 pur (23 mg), 91 pur (19 mg) et une fraction contenant à la fois 90 et 91 non séparés.

92 et 94 sont dissous dans du méthanol à 0°C. On ajoute goutte à goutte, de la soude 4 N, jusqu'à pH basique. Lorsque l'hydrolyse est complète de la résine Dowex 50 H+ est ajoutée de façon à désioniser la solution. On obtient après lyophilisation, les composés 93 (11 mg) et 95 (10 mg). Leurs spectres de R.M.N. confirment leurs structures.

On notera que divers substituants peuvent être indroduits sur le glycoside 89 après l'étape de condensation. Ainsi, dans d'autres essais, les groupes protecteurs ont été éliminés tous ensemble. Cette opération a étalement été effectuée successivement sur les positions 2 et/ou 3 et/ou 4. Des substituants spécifiques ont été introduits, notamment, des groupes $SO_3$. De nombreux glycosides ont été ainsi préparés. On a egalement obtenu d'autres glycosides en utilisant des dérivés d'acide L-iduronique et D-glucuronique comprenant des substituants différents de ceux du dérivé 87.

**Revendications**

1. Dérivés possédant une structure acide uronique, caractérisés en ce qu'ils répondent à la formule III:

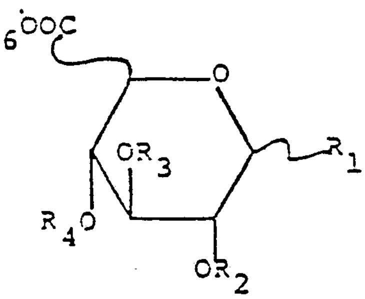

(III)

dans laquelle:

$R_1$ représente un groupe réactif, choisi parmi

> > >. un halogénure, de préférence un chlorure ou un bromure,

. un groupe -O-imidoyle, ou

. conjointement avec le substituant voisin -$OR_2$, un groupe orthoester,
ou encore
. un groupe -O-alcényle, de préférence -O-allyle,
ou
. un groupe -OH libre ou bloqué par un groupement protecteur choisi parmi les radicaux aliphatiques ou aromatiques, en particulier, dans le groupe comprenant un radical alcoyle de 1 à 4 atomes de carbone, tel que méthyle, un radical alcoyle substitué, tel que benzyle, un radical acyle tel qu'acétyle, benzoyle ou chloroacétyle, ou un radical alcenyle de 1 à 4 atomes de carbone, tel qu'allyle ou vinyle, étant entendu que lorsque $R_1$ représente un groupe méthoxy, $OR_4$ est différent d'un tel groupe,

$R_2$, $R_3$ et $R_4$ qui, dans le cas de dérivés glucuroniques ne peuvent être tous trois identiques, $R_3$ et $R_4$ étant en outre différents d'un qroupe acétyle, lorsque $R_1$ et $R_2$ forment conjointement un orthoester, $R_2$ et $R_3$ étant différents d'un groupe benzyle, lorsque $R_1$ représente un atome de chlore et $R_4$ représente un groupe méthyle, ou lorsque $R_1$ représente un groupe -O-alcoyle et $R_4$ un atome d'hydrogène,
représentent
. un groupe alcényle, de préférence un groupe allyle,
. un atome d'hydrogène ou
. un groupe OH bloqué par un groupement protecteur tel que défini ci-dessus pour $R_1$,

$R_6$ représente un atome d'hydrogène ou un groupe protecteur tel que défini ci-dessus, ou les sels de ces dérivés,

à l'exclusion des dérivés d'acide iduronique dans lesquels $R_1$, $R_2$ et $R_3$ représentent tous trois un groupe benzyle et $R_6$ représente un groupe méthyle.

2. Dérivés selon la revendication 1, caractérisés en ce que $R_2$ et $R_3$, et le cas échéant $R_4$, représentent des groupes protecteurs différents, et éliminables séquentiellement sans altération de la structure uronique et de la fonction carboxyle.

3. Dérivés selon la revendication 1, caractérisés en ce qu'ils comportent un groupe réactif en position 1.

4. Dérivés selon la revendication 1, caractérisés par le fait qu'il s'agit de dérivés d'acide D-glucuronique de formule IV

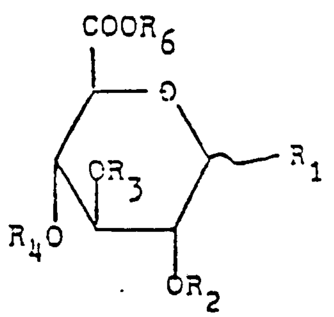

(IV)

dans laquelle:

$R_1$ représente un groupe reactif choisi parmi
. un halogénure, de préférence un chlorure ou un bromure,
. un groupe -O-imidoyle ou,
. conjointement avec $OR_2$, un groupe orthoester,
. un radical -O-alcoyle-substitué ou encore un radical alcényle, de préférence -O-allyle,
. un groupe -OH,

$R_2$ à $R_4$ ne pouvant être tous trois identiques, représentent un groupe protecteur choisi parmi un radical alcoyle de 1 à 3 atomes de carbone, de préférence méthyle, alcoyle substitué, de préférence benzyle, acyle, de préférence acétyle, benzoyle ou chloroacétyle, ou alcényle, de préférence allyle ou vinyle,

$R_6$ représente un radical aliphatique ou aromatique, en particulier un radical alcoyle de 1 à 4 atomes de carbone, ou alcoyle substitué, tel que benzyle, et les sels de ces dérivés.

5. Dérivés selon la revendication 4, caractérisée en ce qu'au moins l'un des groupes $R_2$ ou $R_3$ représente un groupe acyle.

6. Dérivés selon la revendication 4 ou 5, caractérisés en ce que $R_4$ représente un groupe temporaire formant un ester ou un éther avec le groupe -OH qu'il protège, ce groupe temporaire étant choisi parmi les radicaux monochloroacétique, acétyle, benzyle ou p-méthoxybenzyle, allyle, vinyl, et $R_1$ représente un groupe allyle ou vinyle ou un groupe -OH.

7. Dérivés selon la revendication 4 ou 5, caractérisés en ce que $R_1$ représente un groupe -OH ou bien $R_4$ représente un groupe -OH et $R_1$ un groupe alcényle tel que O-allyle.

8. Dérivés selon la revendication 1, caractérisés en ce qu'il s'agit de dérivés de l'acide L-iduronique de formule V

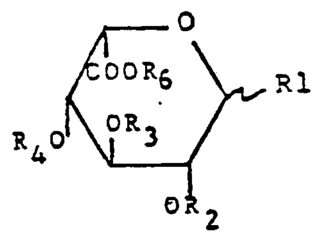

(V)

dans laquelle $R_1$ à $R_5$ présentent les significations données dans la revendication 1.

9. Dérivés selon la revendication 8, caractérisés en ce que $R_1$ et $-OR_2$ représentent conjointement un orthoester, ou bien que $R_1$ est choisi parmi

. un halogénure,

. un radical alcoyle

. ou un radical alcoxyle.

10. Dérivés selon la revendication 9, caractérisés en ce que $R_4$ est un groupe temporaire possédant les significations données dans la revendication 6, et $R_2$ et $R_3$ sont différents l'un de l'autre.

11. Procédé pour la préparation d'un cycle idopyranoside, caractérisé en ce qu'il comprend les étapes suivantes:

- on traite un dérivé d'α-D-glucofuranoside de formule VI:

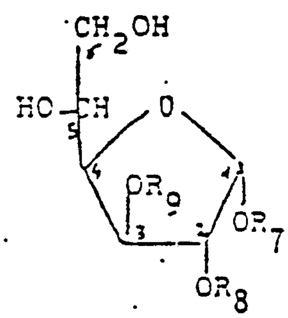

(VI)

dans laquelle les groupes $R_7$ à $R_9$ peuvent être identiques ou différents les une des autres et représentent des groupes de blocage de radicaux hydroxyle tels que définis dans la revendication 1, $R_7$ et $R_8$ formant, de préférence, un groupe isopropylidène et $R_9$ un groupe benzyle,

- on effectue la conversion de la structure D-gluco en position 5, en une structure L-ido,
- on effectue le passage de la structure alcool primaire en position 6 en une structure acide carboxylique,
- on procède au réarrangement de la forme furanose en la forme pyranose.

12. Procédé selon la revendication 11, caractérisé en ce que le traitement des groupes $-CH_2OH$ et -CHOH en positions respectivement 6 et 5 de la structure furanose comprend:

1 - le blocage sélectif du groupe -OH en position 6, en utilisant du chlorure de trityle,

2 - le blocage sélectif du groupe -OH de -CHOH en position 5, avantageusement par un groupe protecteur temporaire tel que benzoyle,

3 - le déblocage sélectif du groupe -OH en position 6 afin d'obtenir un groupe $-CH_2OH$.

4 - l'oxydation du groupe - $CH_2OH$ en un groupe -COOH, en utilisant un agent d'oxydation tel que de l'oxyde chromique,

5 et 6 - le déblocage du groupe -OH en position 5 par traitement par de la soude et la transformation, si souhaitée, du groupe -COOH en position 6 en un groupe -COOMe, en utilisant un agent d'estérification approprié, tel que le diazométhane,

7 - l'introduction en position 5 d'un groupe réactif tel qu'un tosylate, un mésylate, un groupe triflyle,

8 - l'inversion de configuration du carbone en position 5, à l'aide d'un agent nucléophile,

9 - l'élimination du groupe fonctionnel en position 5, à l'aide de méthanol suivie avantageusement d'une chromatographie aux fins de purification,

10 - le réarrangement du cycle pour passer d'un cycle idofuranosique à un cycle idopyranosique en opérant en milieu acide.

13. Procédé selon la revendication 12, caractérisé en ce que l'introduction d'un groupe réactif selon l'étape 7 est réalisée à l'aide d'un dérivé triflique, mésylique ou tosylique.

14. Procédé selon la revendication 13, caractérisé en ce qu'on utilise un dérivé triflique constitué par de de l'anhydride triflique.

15. Procédé selon la revendication 12, caractérisé en ce que l'inversion de configuration selon l'étape 8, est réalisée par substitution nucléophile avec du trifluoracétate de sodium.

16. Application des dérivés uroniques selon l'une quelconque des revendications 1 à 10, à la synthèse de

glycosides utilisables en biologie.

17. Application selon la revendication 16, pour préparer des substrats radioactifs, comprenant la fixation d'un dérivé d'acide uronique à un glycoside radioactif ou à un glycoside traité, après l'étape de condensation du dérivé uronique avec un aglycone, afin d'être radioactif.

18. Application selon la revendication 16, pour préparer des antigènes artificiels, et par là, des anticorps spécifiques, ainsi que des immunoabsorbants, comprenant la fixation sur un support soluble ou insoluble d'un dérivé d'acide uronique, ou d'un glycoside de ce dérivé, ce dérivé d'acide uronique et ce glycoside possédant un bras d'ancrage.

19. Application selon la revendication 17, caractérisé en ce que le bras d'ancrage comprend:

> > >1/ un radical alcoyle de 2 à 10 atomes de carbone, comprenant en bout de chaine, un groupe -OH, le cas échéant protégé ou engagé dans un groupe fonctionnel,

2/ un radical alcényle comprenant de 2 à 10 atomes de carbone et correspondant avantageusement aux radicaux alcoylène-glycol, α,β- hydroxypropyle, ou β-hydroxyéthyle, γ-hydroxypropyle, lesdites chaînes 1 et 2, contenant avantageusement en outre un ou plusieurs groupes éther et/ou amine intercalaire et/ou un groupe fonctionnel terminal comprenant de l'azote tel qu'un groupe amino ou encore un groupe terminal éther, carboxyle ou aldéhyde.

## Patentansprüche

1. Eine Uronsäurestruktur aufweisende Derivate, dadurch gekennzeichnet, daß sie der Formel III:

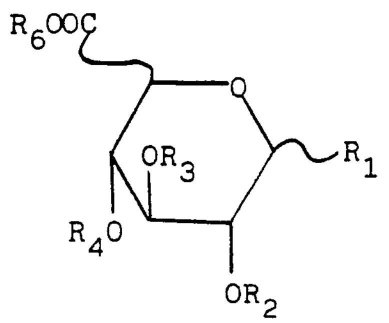

(III)

entsprechen, in welcher:

$R_1$ eine reaktive Gruppe, ausgewählt aus
einem Halogen, vorzugsweise Chlor oder Brom,
einer -O-Imidoylgruppe, oder,
zusammen mit dem Nachbarsubstituenten $-OR_2$, aus einer Orthoestergruppe ausgewählt ist,
oder auch
eine -O-Alkenyl-, vorzugsweise -O-Allyl-, oder
eine freie oder durch eine Schutzgruppe blockierte -OH-Gruppe bedeutet, welche Schutzgruppe aus aliphatischen oder aromatischen Resten, insbesondere aus der Gruppe, umfassend einen Alkylrest mit 1 bis 4 Kohlenstoffatomen, wie Methyl, einen substituierten Alkylrest, wie Benzyl, einen Acylrest, wie Acetyl, Benzoyl oder Chloracetyl, oder einen 1 bis 4 Kohlenstoffatome aufweisenden Alkenylrest, wie Allyl oder Vinyl, ausgewählt ist, mit der Maßgabe, daß, falls $R_1$ eine Methoxygruppe bedeutet, $OR_4$ von einer solchen Gruppe verschieden ist,

$R_2$, $R_3$ und $R_4$, welche im Falle von Glucuronsäurederivaten nicht alle drei identische Reste sein können, wobei $R_3$ und $R_4$ im übrigen von einer Acetylgruppe verschieden sind, falls $R_1$ und $R_2$ gemeinsam einen Orthoester bilden, $R_2$ und $R_3$ von einer Benzylgruppe verschieden sind, falls $R_1$ ein Chloratom bedeutet, und $R_4$ eine Methylgruppe bedeutet,
oder, falls $R_1$ eine -O-Alkylgruppe und $R_4$ ein Wasserstoffatom bedeuten,
eine Alkenylgruppe, vorzugsweise eine Allylgruppe,
ein Wasserstoffatom oder
eine durch eine Schutzgruppe, wie sie oben für $R_1$ definiert ist, blockierte OH-Gruppe bedeuten,

$R_6$ ein Wasserstoffatom oder eine wie oben definierte Schutzgruppe oder die Salze dieser Derivate bedeutet, unter Ausschluß von Iduronsäurederivaten, in welchen alle drei Reste $R_1$, $R_2$ und $R_3$ eine Benzylgruppe bedeuten, und $R_6$ eine Methylgruppe bedeutet.

2. Derivate nach Anspruch 1, dadurch gekennzeichnet, daß $R_2$ und $R_3$, und gegebenenfalls $R_4$, verschiedene und aufeinanderfolgend ohne Anderung der Uronsäurestruktur und der Carboxylfunktion eliminierbare Schutzgruppen bedeuten.

3. Derivate nach Anspruch 1, dadurch gekennzeichnet, daß sie eine reaktive Gruppe in Stellung 1 tragen.

4. Derivate nach Anspruch 1, dadurch gekennzeichnet, daß es sich um D-Glucuronsäurederivate der Formel IV

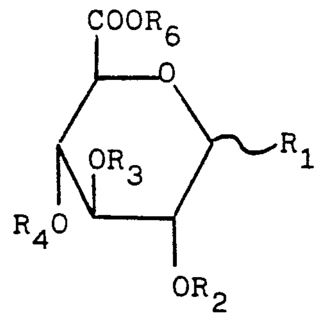

(IV)

handelt, in welcher:

$R_1$ eine reaktive Gruppe, ausgewählt aus
einem Halogen, vorzugsweise Chlor oder Brom,
einer -O-Imidoylgruppe oder,
gemeinsam mit $OR_2$, einer Orthoestergruppe,
einem substituierten -O-Alkylrest, oder auch einem Alkenylrest, vorzugsweise -O-Allyl, und
einer -OH-Gruppe, bedeutet,

$R_2$ bis $R_4$, die nicht alle drei identisch sein können, eine Schutzgruppe bedeuten, die aus einem Alkylrest mit 1 bis 3 Kohlenstoffatomen, vorzugsweise Methyl, substituiertem Alkyl, vorzugsweise Benzyl, Acyl, vorzugsweise Acetyl, Benzoyl oder Chloracetyl, oder Alkenyl, vorzugsweise Allyl oder Vinyl, ausgewählt ist,

$R_6$ einen aliphatischen oder aromatischen Rest, insbesondere einen Alkylrest mit 1 bis 4 Kohlenstoffatomen, oder substituiertes Alkyl, wie Benzyl, und die Salze dieser Derivate, bedeutet.

5. Derivate nach Anspruch 4, dadurch gekennzeichnet, daß wenigstens eine der Gruppen $R_2$ oder $R_3$ eine Acylgruppe bedeutet.

6. Derivate nach Anspruch 4 oder 5, dadurch gekennzeichnet, daß $R_4$ eine temporäre Gruppe bedeutet, welche mit der zu schützenden -OH-Gruppe einen Ester oder einen Ether bildet, wobei diese temporäre Gruppe aus den Monochloressigsäure-, Acetyl-, Benzyl- oder p-Methoxybenzyl-, Allyl- und Vinylresten ausgewählt ist, und $R_1$ eine Allyl- oder Vinylgruppe oder eine -OH-Gruppe bedeutet.

7. Derivate nach Anspruch 4 oder 5, dadurch gekennzeichnet, daß $R_1$ eine -OH-Gruppe bedeutet, oder aber $R_4$ eine -OH-Gruppe bedeutet, und $R_1$ eine Alkenyl-, wie O-Allyl-, -gruppe bedeutet.

8. Derivate nach Anspruch 1, dadurch gekennzeichnet, daß es sich um L-Iduronsäurederivate der Formel V

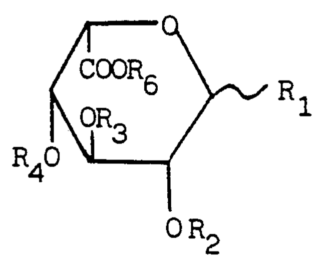

(V)

handelt, in welcher $R_1$ bis $R_5$ die in Anspruch 1 angegebenen Bedeutungen haben.

9. Derivate nach Anspruch 8, dadurch gekennzeichnet, daß $R_1$ und $-OR_2$ zusammen einen Orthoester bedeuten, oder aber $R_1$ aus einem Halogen, einem Alkylrest oder einem Alkoxyrest ausgewählt ist.

10. Derivate nach Anspruch 9, dadurch gekennzeichnet, daß $R_4$ eine temporäre Gruppe ist, welche die in Anspruch 6 angegebenen Bedeutungen hat, und $R_2$ und $R_3$ voneinander verschieden sind.

11. Verfahren zur Herstellung eines Cycloidopyranosids, dadurch gekennzeichnet, daß es die folgenden Stufen umfaßt:

- Behandlung eines α-D-Glucofuranosidderivats der Formel VI:

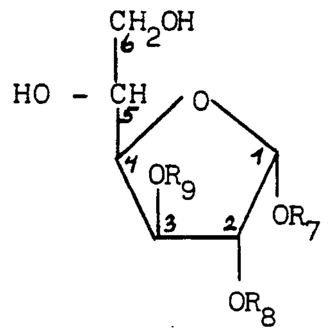

(VI) ,

in welcher die Gruppen $R_7$ bis $R_9$ gleich oder voneinander verschieden sein können, und Blockierungsgruppen von Hydroxylresten bedeuten, wie sie in Anspruch 1 definiert sind, $R_7$ und $R_8$ vorzugsweise eine Isopropylidengruppe bilden, und $R_9$ eine Benzylgruppe bedeutet,

- Umwandlung der D-Gluco-Struktur in Stellung 5 in eine L-Ido-Struktur,
- Überführen der primären Alkoholstruktur in Stellung 6 in eine Carbonsäurestruktur,
- Umlagerung der Furanoseform in die Pyranoseform.

12. Verfahren nach Anspruch 11, dadurch gekennzeichnet, daß die Behandlung der $-CH_2OH$- und -CHOH-Gruppen in den Stellungen 6 bzw. 5 der Furanosestruktur umfaßt:

1 - die selektive Blockierung der -OH-Gruppe in Stellung 6 unter Verwendung von Tritylchlorid,

2 - die selektive Blockierung der -OH-Gruppe von -CHOH in Stellung 5, vorteilhafterweise durch eine temporäre Schutzgruppe, wie Benzoyl,

3 - die selektive Entblockierung der -OH-Gruppe in Stellung 6, um eine $-CH_2OH$-Gruppe zu erhalten,

4 - die Oxydation der Gruppe $-CH_2OH$ in eine -COOH-Gruppe unter Verwendung eines Oxydationsmittels, wie Chromoxid,

5 und 6 - die Entblockierung der -OH-Gruppe in Stellung 5 durch Behandlung mit Alkali und gewünschtenfalls die Umwandlung der -COOH-Gruppe in Stellung 6 in eine -COOMe-Gruppe, unter Verwendung eines entsprechenden Veresterungsmittels, wie Diazomethan,

7 - die Einführung einer reaktiven Gruppe, wie eines Tosylats, eines Mesylats, oder einer Trifluoracetylessigsäuregruppe, in Stellung 5,

8 - die Inversion der Konfiguration des Kohlenstoffs in Stellung 5 mit Hilfe eines nukleophilen Mittels,

9 - die Eliminierung der funktionellen Gruppe in Stellung 5 mit Hilfe von Methanol, und vorteilhafterweise anschließend eine Chromatographie zum Zwecke der Reinigung,

10 - die Ringumlagerung, um einen Idofuranosering in einen Idopyranosering umzuwandeln, wobei in saurem Milieu gearbeitet wird.

13. Verfahren nach Anspruch 12, dadurch gekennzeichnet, daß die Einführung einer reaktiven Gruppe gemäß Stufe 7 mit Hilfe eines Trifluoressigsäure-, Methansulfonsäure- oder Toluolsulfonsäurederivats bewirkt wird.

14. Verfahren nach Anspruch 13, dadurch gekennzeichnet, daß man ein Trifluoressigsäurederivat verwendet, das aus dem Anhydrid der Trifluoressigsäure besteht.

15. Verfahren nach Anspruch 12, dadurch gekennzeichnet, daß man die Inversion der Konfiguration gemäß Stufe 8 durch nukleophile Substitution mit trifluoressigsaurem Natrium bewirkt.

16. Anwendung der Uronsäurederivate nach einem der Ansprüche 1 bis 10, bei der Synthese von biologisch verwendbaren Glykosiden.

17. Anwendung nach Anspruch 16 zur Herstellung von radioaktiven Substraten, umfassend die Fixierung eines Uronsäurederivats an einem radioaktiven Glykosid, oder an einem Glykosid, das nach der Stufe der Kondensation des Uronsäurederivats mit einem Aglykon zur Schaffung von Radioaktivität behandelt worden ist.

18. Anwendung nach Anspruch 16 zur Herstellung von künstlichen Antigenen und, davon ausgehend, von spezifischen Antikörpern, sowie von Immun absorbenten umfassend die Fixierung eines Uronsäurederivats oder eines Glykosids dieses Derivates auf einem löslichen oder unlöslichen Träger, wobei dieses Uronsäurederivat und dieses Glykosid einen Verankerungsarm aufweisen.

19. Anwendung nach Anspruch 17, dadurch gekennzeichnet, daß dieser Verankerungsarm umfaßt:

1. einen Alkylrest mit 2 bis 10 Kohlenstoffatomen, der am Ende der Kette eine -OH-Gruppe enthält, welche gegebenenfalls geschützt oder in eine funktionelle Gruppe einbezogen ist,

2. einen Alkenylrest mit 2 bis 10 Kohlenstoffatomen, der vorteilhafterweise Alkylenglykol-, $\alpha,\beta$-Hydroxypropylen- oder $\beta$-Hydroxyethylen, $\gamma$-Hydroxypropylenresten entspricht, wobei die genannten Ketten 1 und 2 vorteilhafterweise außerdem eine oder mehrere dazwischenliegende Ether- und/oder Amingruppen und/oder eine endständige, funktionelle, stickstoffhältige Gruppe, wie eine Aminogruppe oder auch eine endständige Ether-, Carboxyl- oder Aldehydgruppe enthalten.

## Claims

1. Derivatives possessing a uronic acid structure, characterised in that they correspond to formula III:

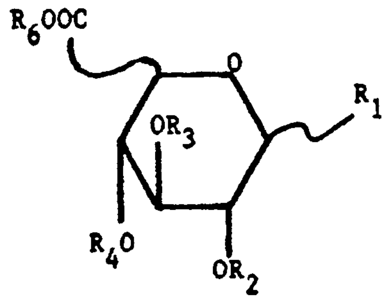

(III)

in which

$R_1$ represents a reactive group selected from
  . a halide, preferably a chloride or a bromide`
  . a -O-imidoyl group, or
  . together with the adjacent $-OR_2$ substituant, an orthoester group,
or again
  . a -O-alkenyl group, preferably -O-allyl,
or
  . an OH group which is free or is blocked by a protective`grouping selected from the aliphatic or aromatic radicals, in particular in the group comprising an alkyl radical containing from 1 to 4 carbon atoms such as methyl, a substituted alkyl radical such as benzyl, an acyl such as acetyl, benzoyl or chloroacetyl, or an alkenyl radical containing from 1 to 4 carbon atoms such as allyl or vinyl, assuming that, when $R_1$ represents a methoxy group, $OR_4$ is different from such a group,

$R_2$, $R_3$ and $R_4$, the three of which cannot all be identical in the case of glucuronic derivatives, $R_3$ and $R_4$ also being different from an acetyl group when $R_1$ and $R_2$ together form an orthoester, $R_2$ and $R_3$ being different from a benzyl group when $R_1$ represents a chlorine atom and $R_4$ represents a methyl group, or when $R_1$ represents a -O-alkyl group and $R_4$ a hydrogen atom, represent
  . an alkenyl group, preferably an allyl group,`
  . a hydrogen atom or
  . an OH group blocked by a protective grouping as defined above for $R_1$,

$R_6$ represents a hydrogen atom or a protective group as defined above, or the salts of these derivatives, with the exclusion of iduronic acid derivatives in which $R_1$, $R_2$ and $R_3$ all represent a benzyl group and $R_6$ represents a methyl group.

2. Derivatives according to claim 1, characterised in that $R_2$ and $R_3$, and optionally $R_4$, represent different protective groups which can be eliminated sequentially without altering the uronic structure and the carboxyl function.

3. Derivatives according to claim 1, characterised in that they comprise a reactive group in position 1.

4. Derivatives according to claim 1, characterised in that they are D-glucuronic acid derivatives corresponding to formula IV

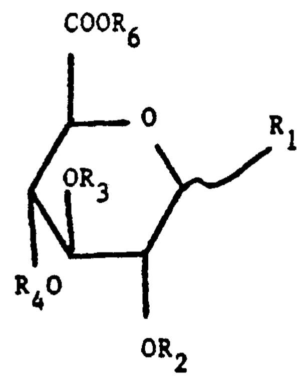

(IV)

in which

$R_1$ represents a reactive group selected from
  . a halide, preferably a chloride or bromide,`
  . a -O-imidoyl group or,
  . together with $OR_2$, an orthoester group,
  . a substituted -O-alkyl radical or again an alkenyl radical preferably -O-allyl,
  . an -OH group,

$R_2$ to $R_4$, which cannot all be identical, represent a protective group selected from an alkyl radical containing from 1 to 3 carbon atoms, preferably methyl, substituted alkyl, preferably benzyl, acyl, preferably acetyl, benzoyl or chloroacetyl, or alkenyl, preferably allyl or vinyl,

$R_6$ represents an aliphatic or aromatic radical, in particular an alkyl radical containing from 1 to 4 carbon atoms, or a substituted alkyl radical such as benzyl, and the salts of these derivatives.

5. Derivatives according to claim 4, characterised in that at least one of the groups $R_2$ or $R_3$ represents an acyl group.

6. Derivatives according to claim 4 or 5, characterised in that $R_4$ represents a temporary group forming an ester or an ether with the group -OH which it protects, this temporary group being selected from the monochloroacetic, acetyl, benzyl or p-methoxybenzyl, allyl and vinyl radicals and $R_1$ represents an allyl or vinyl group or a -OH group.

7. Derivatives according to claim 4 or 5, characterised in that $R_1$ represents a -OH group or $R_4$ represents a -OH group and R1 represents an alkenyl group such as O-allyl.

8. Derivatives according to claim 1, characterised in that they are derivatives of L-iduronic acid corresponding to formula V

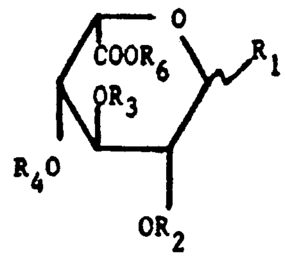

(V)

in which $R_1$ to $R_5$ have the meanings given in claim 1.

9. Derivatives according to claim 6, characterised in that $R_1$ and $-OR_2$ together represent an orthoester or $R_1$ is selected from

. a halide,
. an alkyl radical
. or an alkoxyl radical.

10. Derivatives according to claim 9, characterised in that $R_4$ is a provisional group having the meanings given in claim 6, and $R_2$ and $R_3$ are different from one another.

11. A process for the preparation of an idopyranoside cycle, chatacterised in that it comprises the following stages:

- a derivative of α-D-glucofuranoside corresponding to formula VI

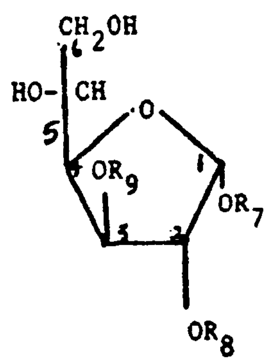

(VI)

in which the groups $R_7$ to $R_9$ can be identical to or different from one another and represent groups for blocking hydroxyl radicals as defined in claim 1, $R_7$ and $R_8$ preferably forming an isopropylidene group and $R_9$ a benzyl group, is treated,

- the D-gluco structure in position 5 is converted into a' L-ido structure
- the primary alchohol structure in position 6 is passed into a carboxylic acid structure,
- the furanose form is rearranged into the pyranose form.

12. A process according to claim 11, characterised in that the treatment of the $-OH_2OH$ and -CHOH groups in positions 6 and 5 respectively of the furanose structure comprises:

1 - selective blocking of the -OH group in position 6, using' trityl chloride,

2 - selective blocking of the -OH group of -CHOH in position 5, advantagpously by a temporary protective group such as benzoyl,

3 - selective unblocking of the -OH group in position 6 in order to obtain a $-CH_2OH$ group,

4 - oxidation of the $-CH_2OH$ group into a -COOH group, using an oxidizing agent such as chromic oxide,

5 and 6 - unblocking of the -OH group in position 5 by treatment with soda and transformation, if

desired, of the -COOH group in position 6 into a -COOMe group using a suitable esterification agent such as diazomethane,

7 - introduction in position 5 of a reactive group such as a tosylate, a mesylate, a triflyl group,

8 - inversion of configuration of the carbon in position 5 using a nucleophilic agent,

9 - elimination of the functional group in position 5 using methanol, advantageously followed by chromatography for the purpose of purification,

10 - rearrangement of the cycle so as to pass from an idofuranosic cycle to an idopyranosic cycle while working in an acidic medium.

13. A process according to claim 12, characterised in that a reactive group according to stage 7 is introduced by means of a triflyl, mesyl or tosyl derivative.

14. A process according to claim 13, characterised in that a triflyl derivative constituted by triflyl anhydride is used.

15. A process according to claim 12, characterised in that the inversion of configuration according to stage 8 is carried out by nucleophilic substitution with sodium trifluoroacetate.

16. Use of the uronic derivatives according to any one of claims 1 to 10 for the synthesis of glycosides with can be used in biology.

17. Use according to claim 16, for the preparation of radioactive substrates comprising fixing a uronic acid derivative to a radioactive glycoside or to a glycoside which has been treated, after the stage of condensing the uronic derivative with an aglycon, so as to be radioactive.

18. Use according to claim 16 for the preparation of artifical antigens and, consequently, specific antibodies as well as immuno-absorbents, comprising fixing on a soluble or insoluble support a uronic acid derivative or a glycoside of this derivative, this uronic acid derivative and this glycoside having an anchoring arm.

19. Use according to claim 17, characterised in that the anchoring arm comprises:

1. an alkyl radical containing from 2 to 10 carbon atoms and comprising, at the end of the chain, a -OH group which may optionally be protected or engaged in a functional group.

2. an alkenyl radical comprising from 2 to 10 carbon atoms and advantageously corresponding to the alkylene glycol, α,β-hydroxypropyl, or β-hydroxyethyl, γ-hydroxypropyl radical, said chains 1 and 2 also advantageously containing one or more ether and/or intercalation amine groups and/or a functional end group comprising nitrogen such as an amino group or again an ether, carboxyl or aldehyde end group.

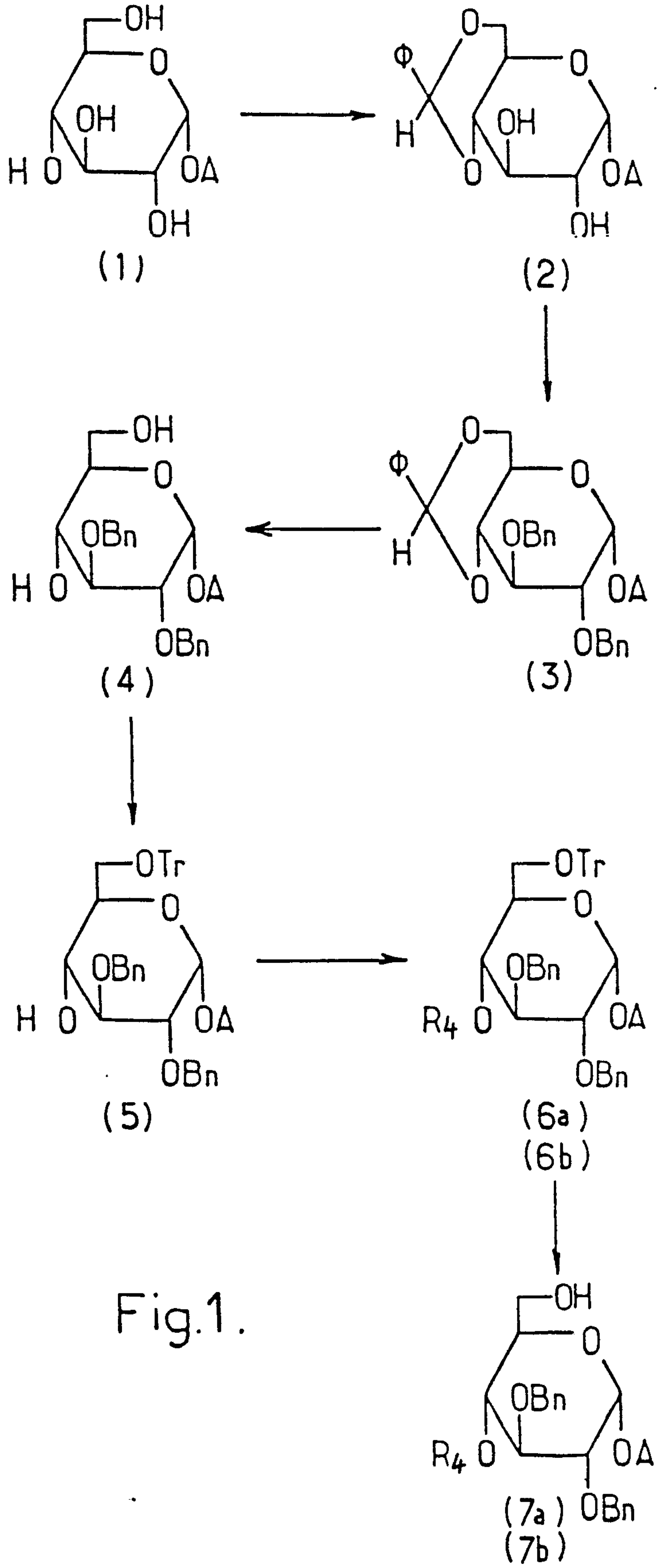

(1)

(2)

(3)

(4)

(5)

(6a)
(6b)

(7a)
(7b)

Fig.1.

Fig.2.

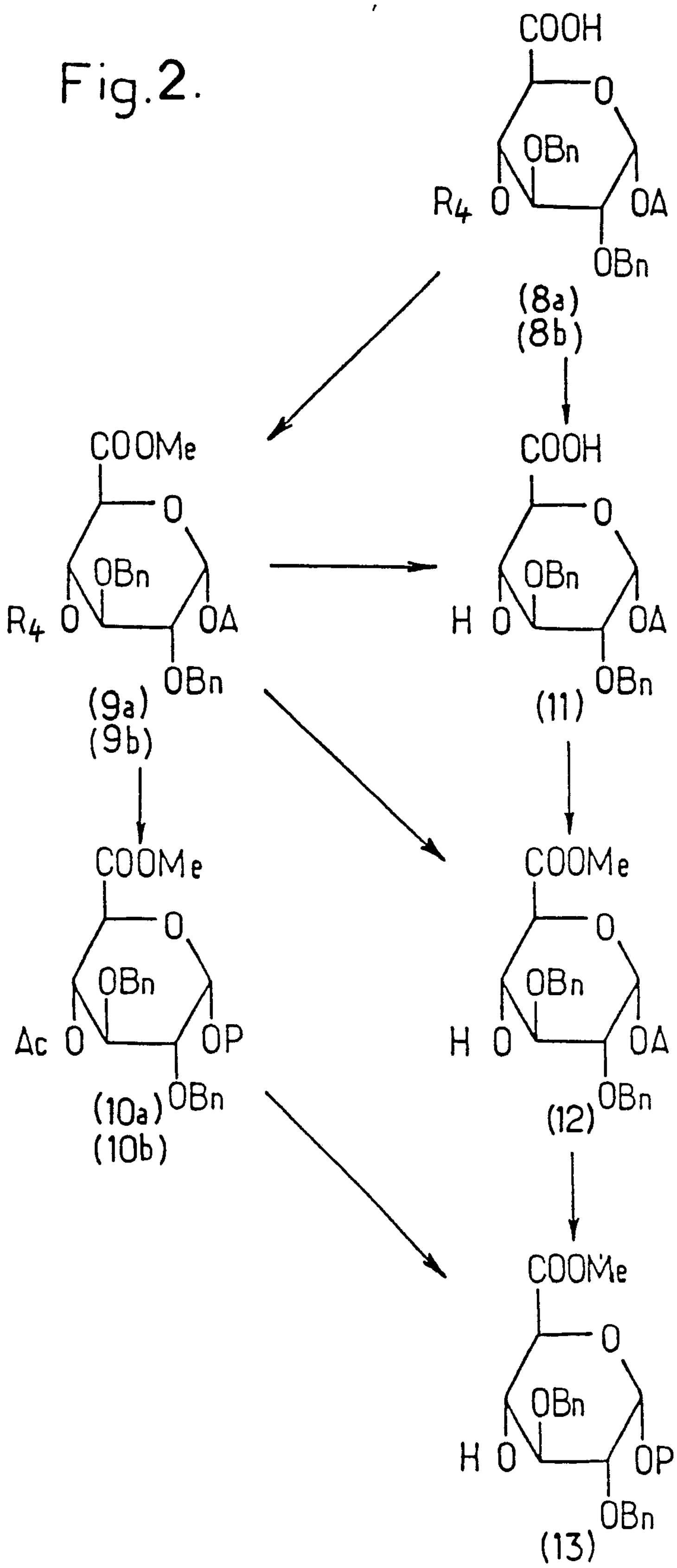

(8a) (8b) (9a) (9b) (10a) (10b) (11) (12) (13)

Fig. 3.

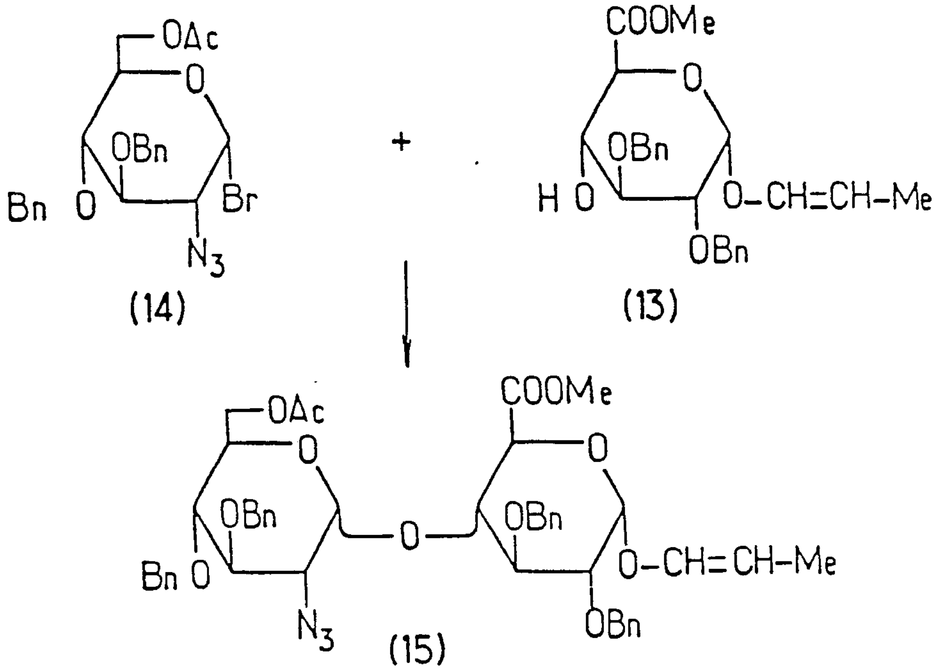

(14) (13)

(15)

Fig. 5.

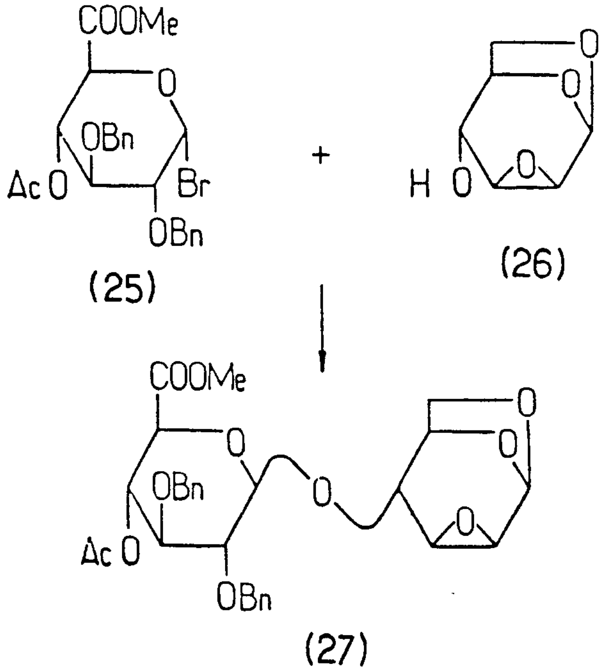

(25) (26)

(27)

Fig.4.

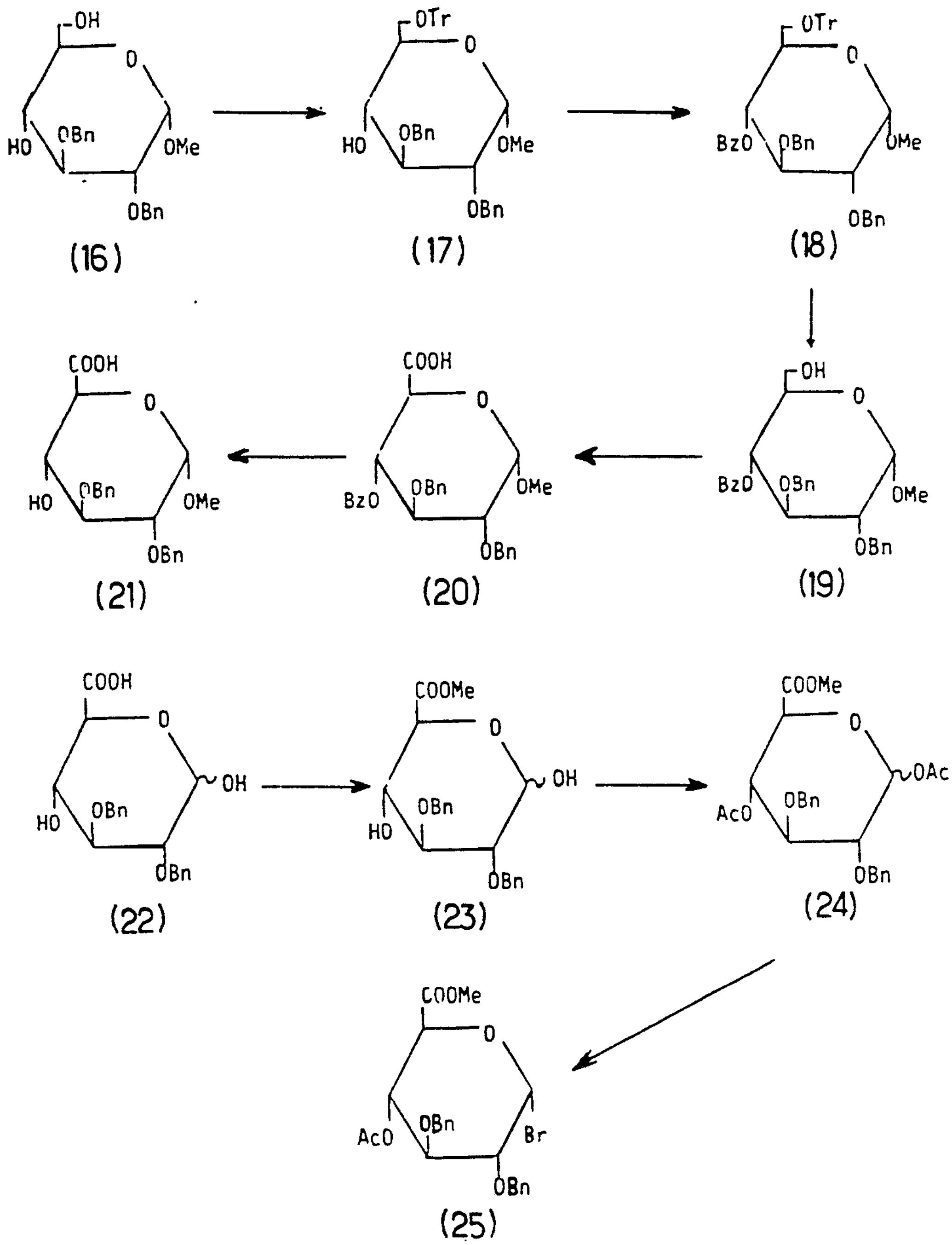

(16) (17) (18)

(21) (20) (19)

(22) (23) (24)

(25)

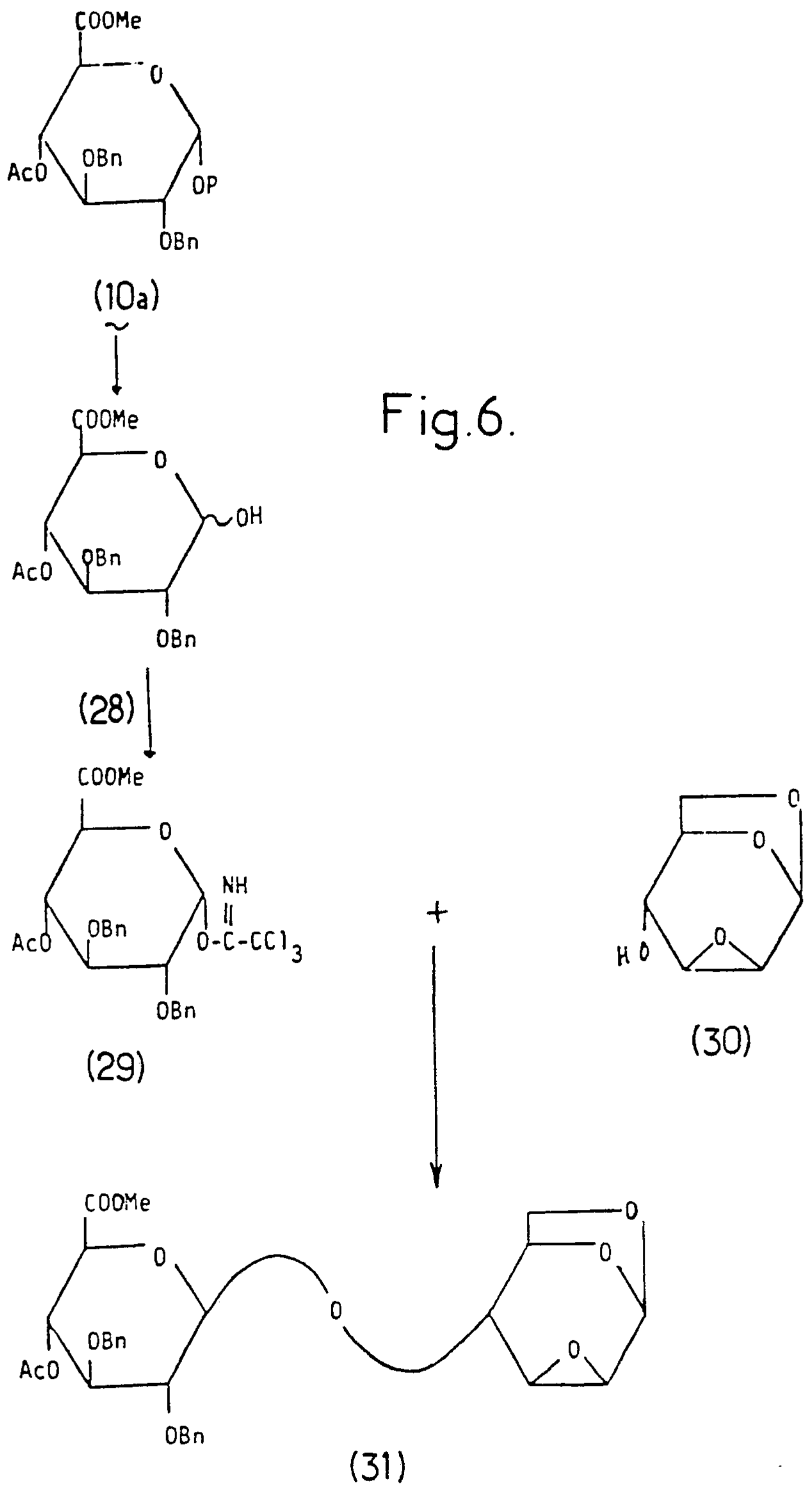

(10a)

(28)

(29)

+

(30)

(31)

Fig.6.

Fig. 7.

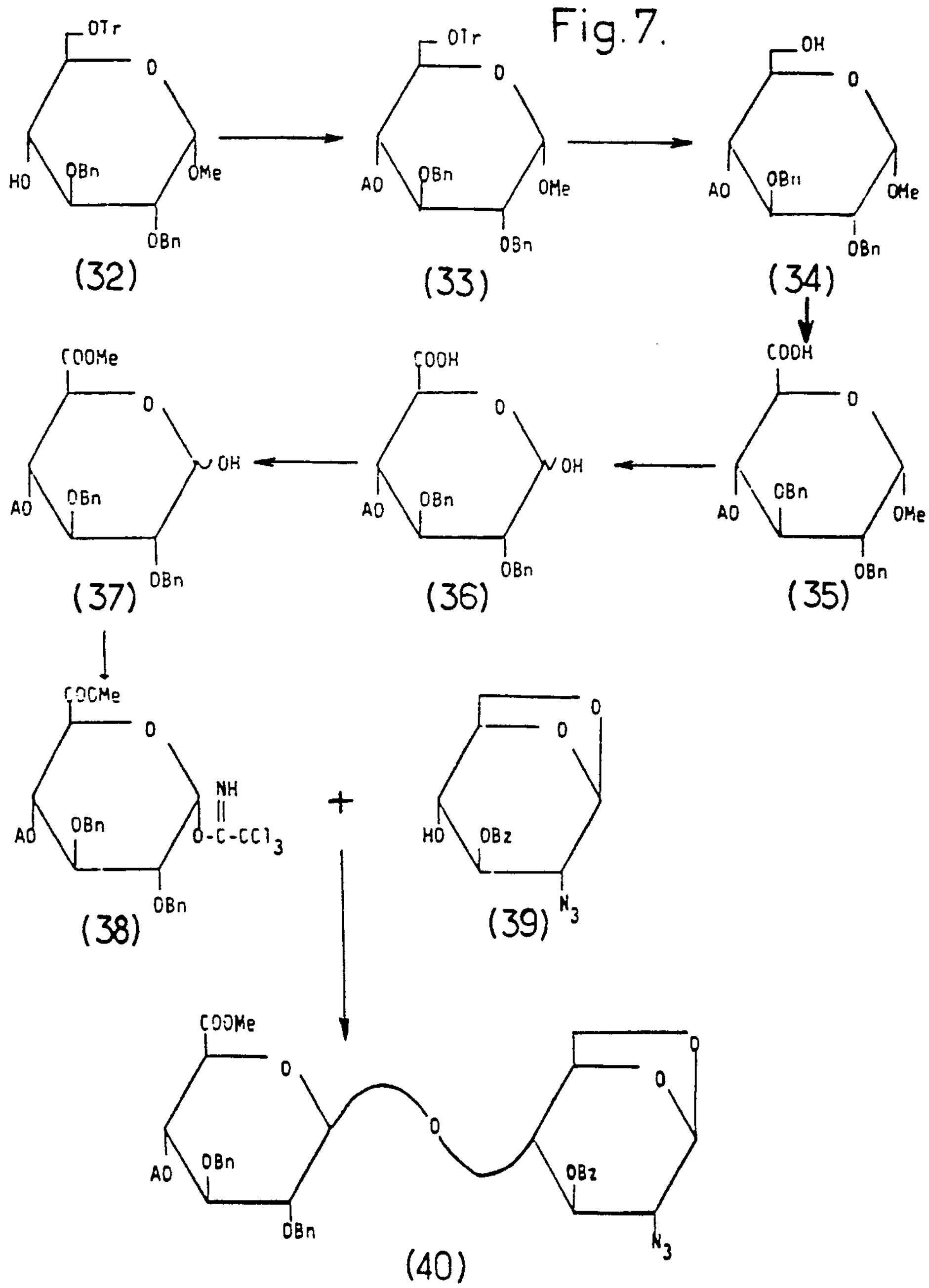

(32) (33) (34) (35) (36) (37) (38) (39) (40)

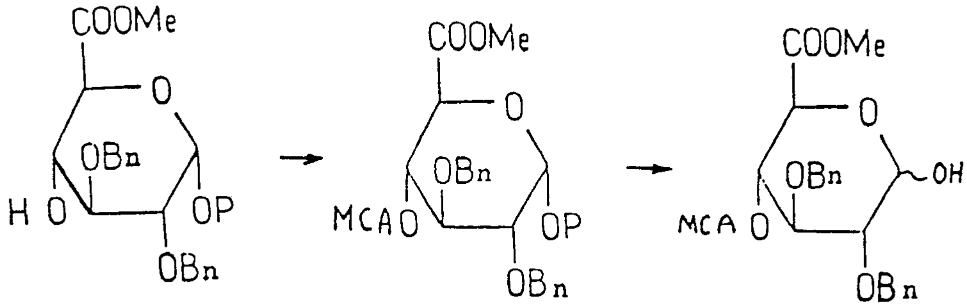

(13) (41) (42)

Fig. 8.

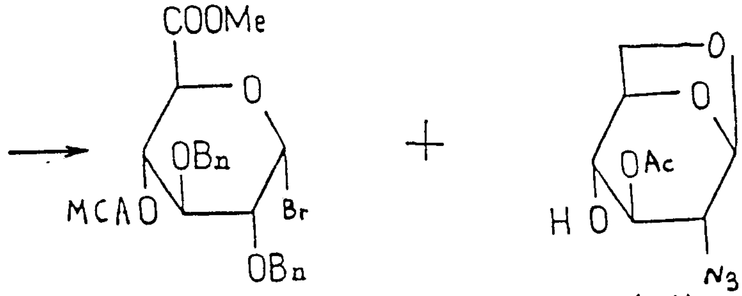

(43) (44)

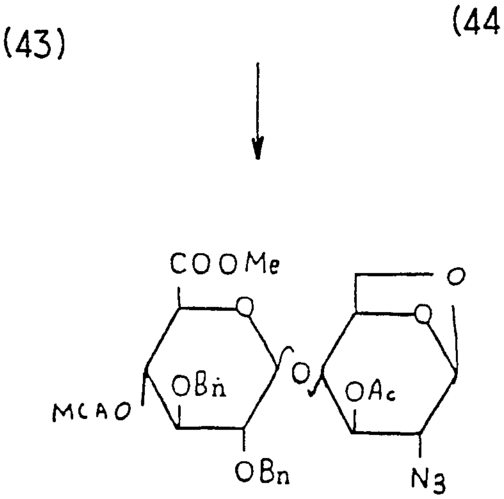

(45)

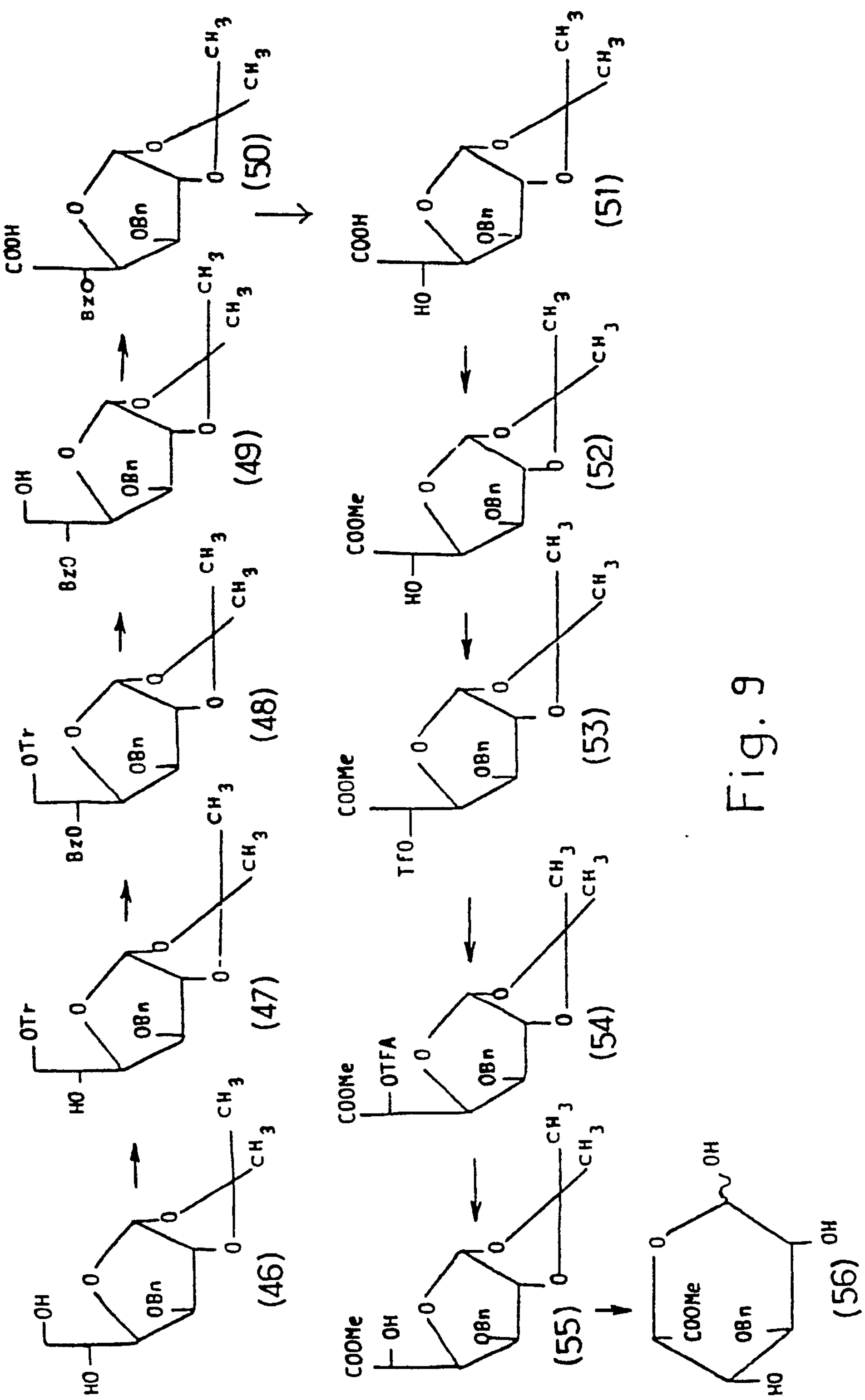

(46) (47) (48) (49) (50) (51) (52) (53) (54) (55) (56)

Fig. 9

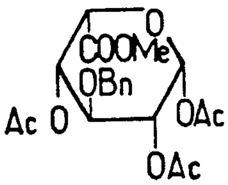

(58)

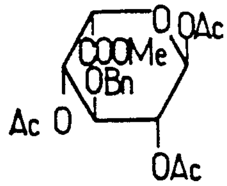

(57)

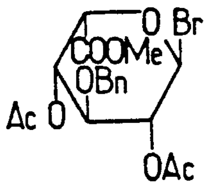

(59)

Fig.10.

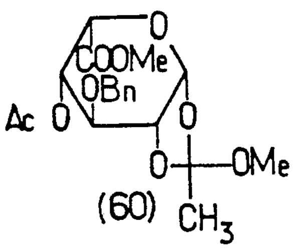

(60)

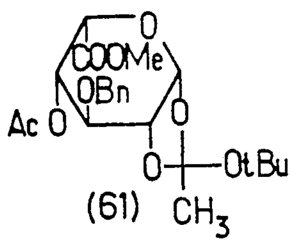

(61)

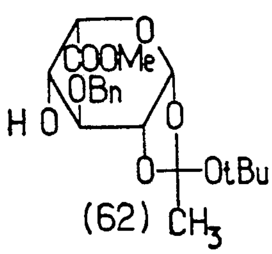

(62)

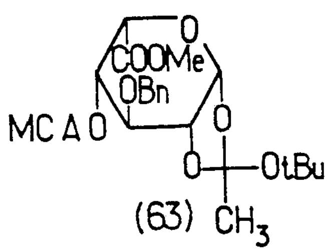

(63)

Fig.11.

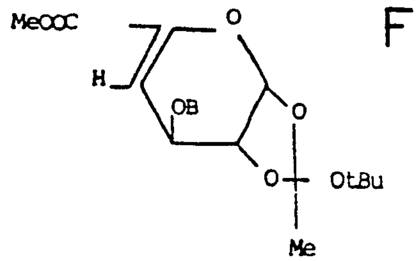

composé (64)

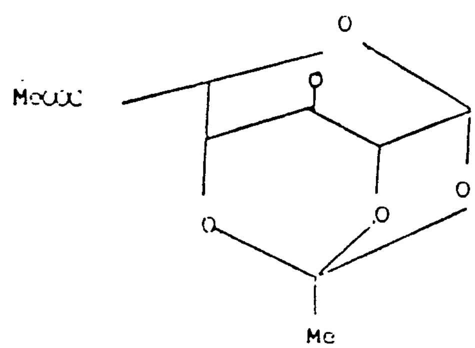

composé (65)

63 +

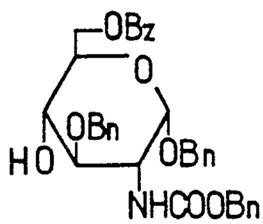

(66)

Fig.12.

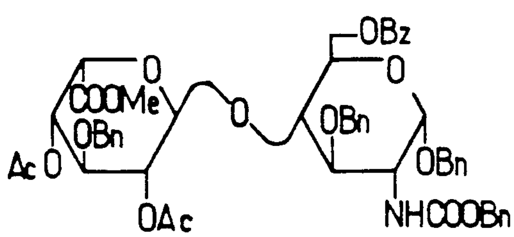

(67)

Fig.13.

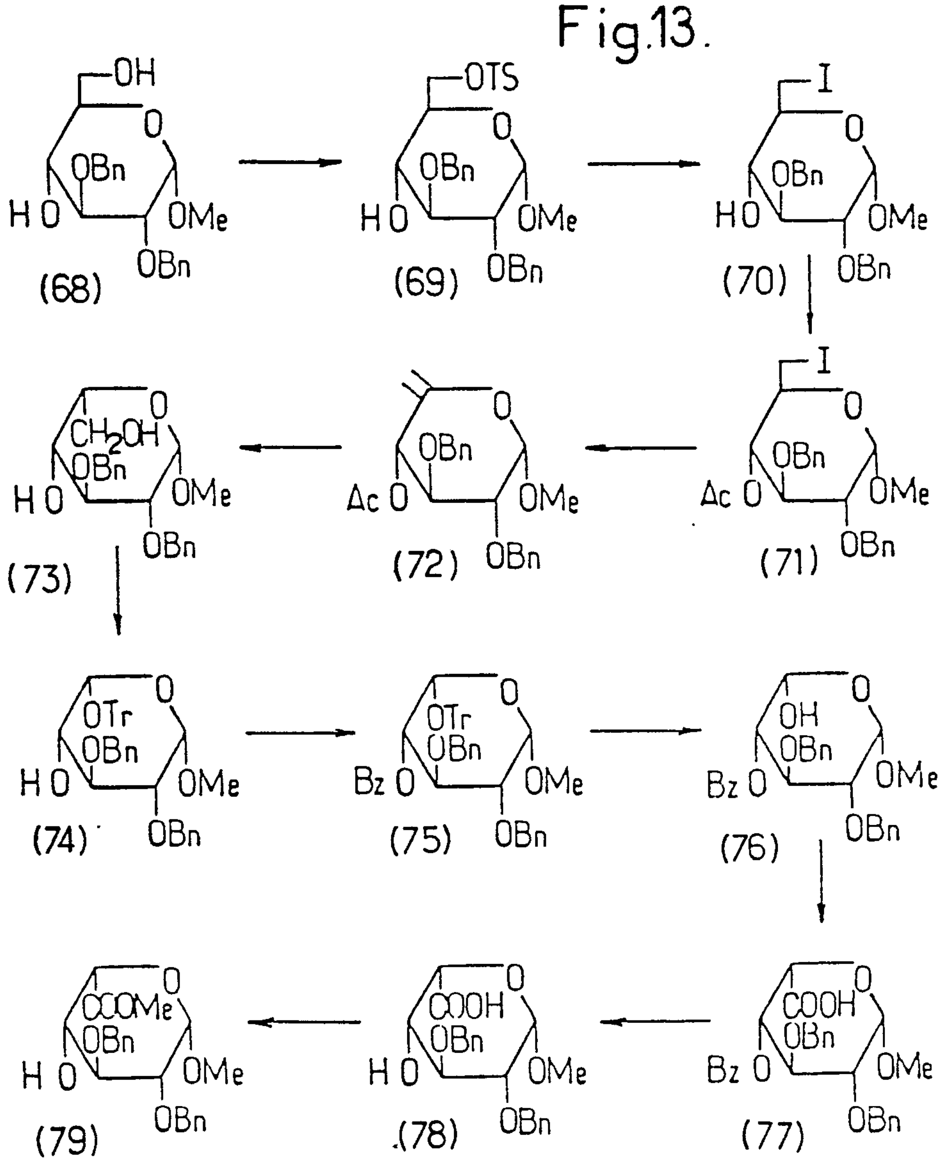

(68) (69) (70)

(73) (72) (71)

(74) (75) (76)

(79) (78) (77)

Fig.14.

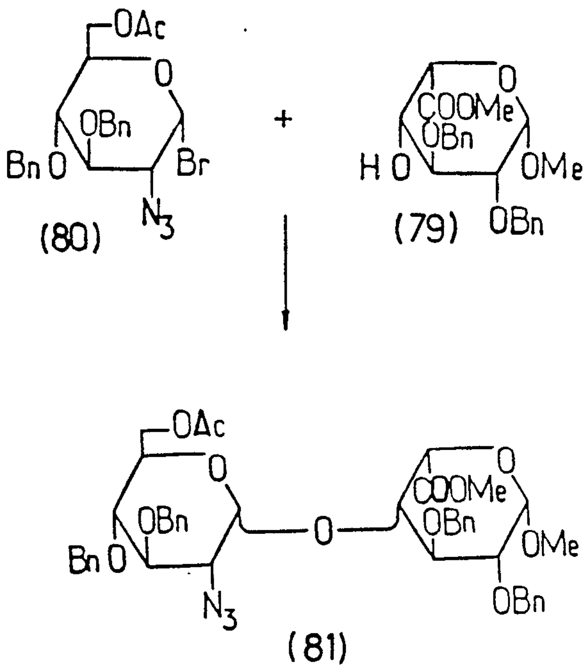

(80) (79)

(81)

Fig.15.

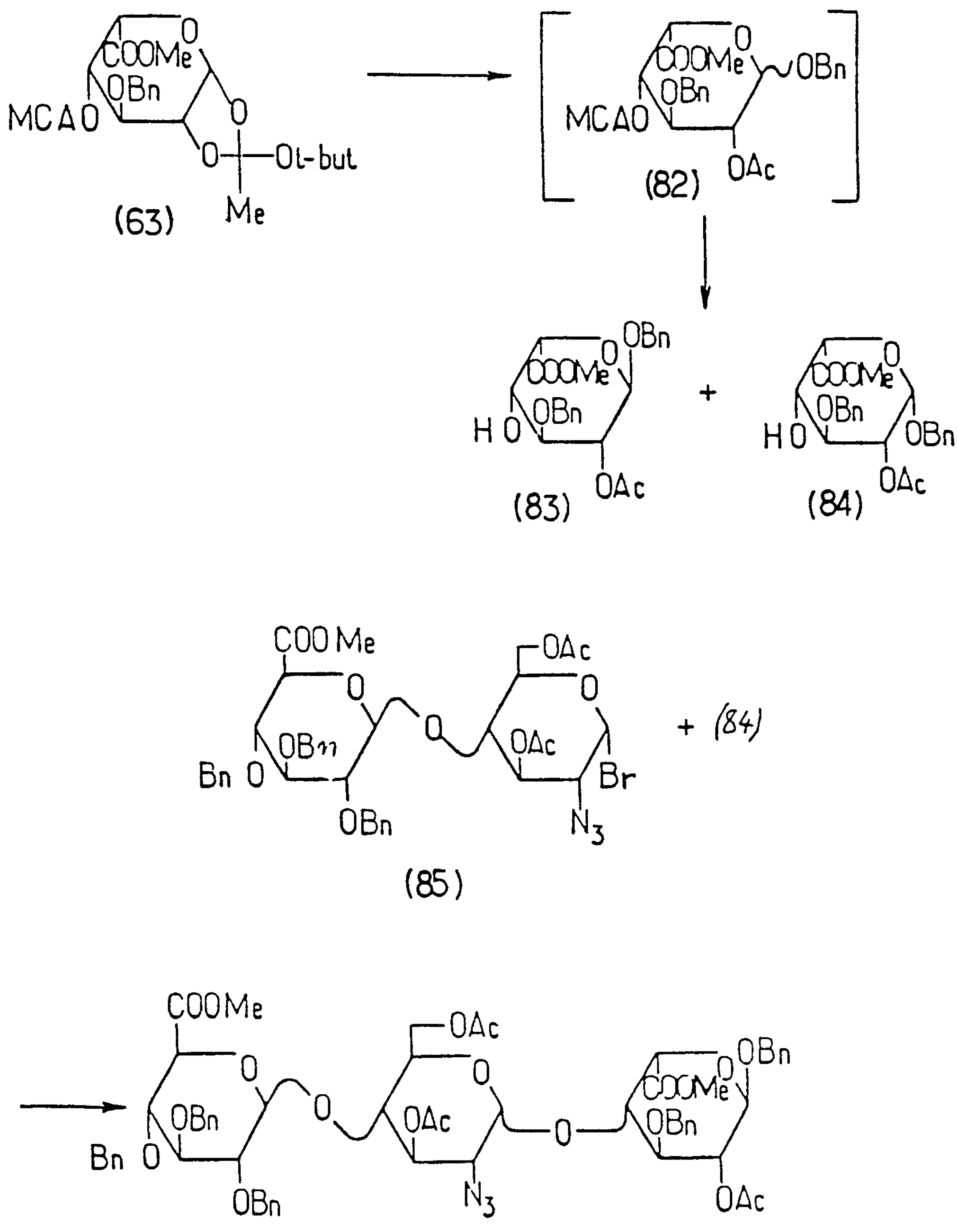

(63) (82) (83) + (84) (85) + (84) (86)

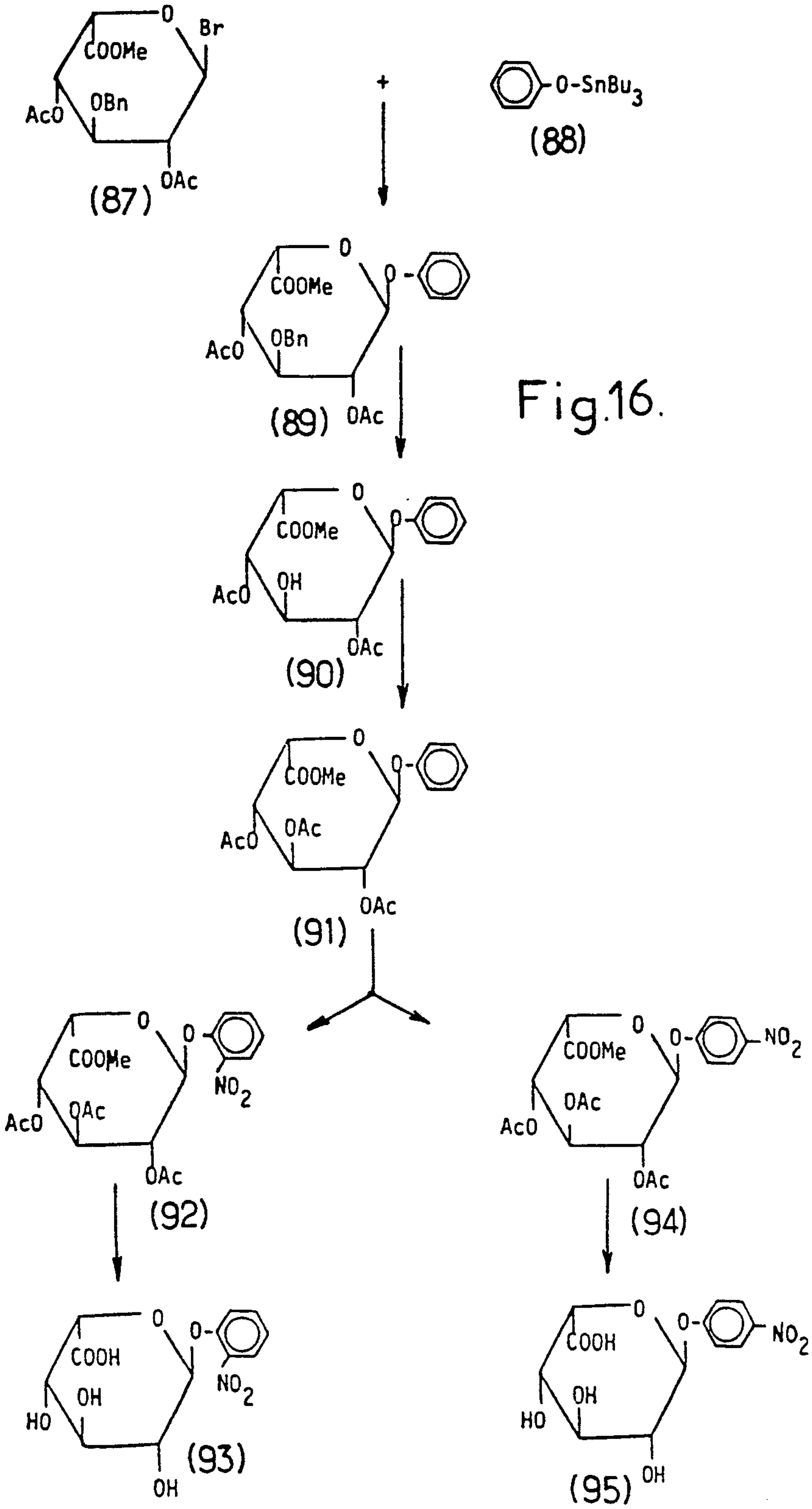

(87) (88) (89) (90) (91) (92) (93) (94) (95)

Fig.16.